# EUROPEAN PATENT APPLICATION

(11) **EP 2 963 108 A1**
(43) Date of publication of application: **06.01.2016**
(21) Application number: 14175042.2
(22) Date of filing: 30.06.2014
(51) Int. Cl.: C12N 5/071

(54) **Methods for inducing insulin production and uses thereof**

(71) Applicant: UNIVERSITE DE GENEVE, 1211 Geneva 4 (CH)
(72) Inventor: Herrera, Pedro L., 1225 Chene-Bourg (CH); Thorel, Fabrizio, 74200 Thonon Les Bains (FR); Chera, Simona, 1205 Geneva (CH)
(74) Representative: reuteler & cie SA

(57) **Abstract**

The invention relates to methods of inducing insulin production in delta-cells and/or converting delta-cells into insulin producing cells, as well as methods of preventing and/or treating diabetes and agents and compositions useful in said methods.

SEQUENCE LISTING

<110>  UNIVERSITE DE GENEVE

<120>  METHODS FOR INDUCING INSULIN PRODUCTION AND USES THEREOF

<130>  P1775EP00

<160>  60

<170>  PatentIn version 3.5

<210>  1
<211>  652
<212>  PRT
<213>  Mus musculus

<400>  1

Met Ala Glu Ala Pro Gln Val Val Glu Thr Asp Pro Asp Phe Glu Pro
1               5                   10                  15

Leu Pro Arg Gln Arg Ser Cys Thr Trp Pro Leu Pro Arg Pro Glu Phe
            20                  25                  30

Asn Gln Ser Asn Ser Thr Thr Ser Ser Pro Ala Pro Ser Gly Gly Ala
        35                  40                  45

Ala Ala Asn Pro Asp Ala Ala Ala Ser Leu Ala Ser Ala Ser Ala Val
    50                  55                  60

Ser Thr Asp Phe Met Ser Asn Leu Ser Leu Leu Glu Glu Ser Glu Asp
65                  70                  75                  80

Phe Ala Arg Ala Pro Gly Cys Val Ala Val Ala Ala Ala Ala Ala Ala
                85                  90                  95

Ser Arg Gly Leu Cys Gly Asp Phe Gln Gly Pro Glu Ala Gly Cys Val
            100                 105                 110

His Pro Ala Pro Pro Gln Pro Pro Pro Thr Gly Pro Leu Ser Gln Pro
        115                 120                 125

Pro Pro Val Pro Pro Ser Ala Ala Ala Ala Ala Gly Pro Leu Ala Gly
    130                 135                 140

Gln Pro Arg Lys Thr Ser Ser Ser Arg Arg Asn Ala Trp Gly Asn Leu
145                 150                 155                 160

Ser Tyr Ala Asp Leu Ile Thr Lys Ala Ile Glu Ser Ser Ala Glu Lys
                165                 170                 175

Arg Leu Thr Leu Ser Gln Ile Tyr Glu Trp Met Val Lys Ser Val Pro
            180                 185                 190

Tyr Phe Lys Asp Lys Gly Asp Ser Asn Ser Ser Ala Gly Trp Lys Asn
        195                 200                 205

Ser Ile Arg His Asn Leu Ser Leu His Ser Lys Phe Ile Arg Val Gln
    210                 215                 220

Asn Glu Gly Thr Gly Lys Ser Ser Trp Trp Met Leu Asn Pro Glu Gly
225                 230                 235                 240

Gly Lys Ser Gly Lys Ser Pro Arg Arg Arg Ala Ala Ser Met Asp Asn
                245                 250                 255

Asn Ser Lys Phe Ala Lys Ser Arg Gly Arg Ala Ala Lys Lys Lys Ala
            260                 265                 270

Ser Leu Gln Ser Gly Gln Glu Gly Pro Gly Asp Ser Pro Gly Ser Gln
        275                 280                 285

Phe Ser Lys Trp Pro Ala Ser Pro Gly Ser His Ser Asn Asp Asp Phe
    290                 295                 300

Asp Asn Trp Ser Thr Phe Arg Pro Arg Thr Ser Ser Asn Ala Ser Thr
305                 310                 315                 320

Ile Ser Gly Arg Leu Ser Pro Ile Met Thr Glu Gln Asp Asp Leu Gly
                325                 330                 335

Asp Gly Asp Val His Ser Leu Val Tyr Pro Pro Ser Ala Ala Lys Met
            340                 345                 350

Ala Ser Thr Leu Pro Ser Leu Ser Glu Ile Ser Asn Pro Glu Asn Met
        355                 360                 365

Glu Asn Leu Leu Asp Asn Leu Asn Leu Leu Ser Ser Pro Thr Ser Leu
    370                 375                 380

Thr Val Ser Thr Gln Ser Ser Pro Gly Ser Met Met Gln Gln Thr Pro
385                 390                 395                 400

Cys Tyr Ser Phe Ala Pro Pro Asn Thr Ser Leu Asn Ser Pro Ser Pro
                405                 410                 415

Asn Tyr Ser Lys Tyr Thr Tyr Gly Gln Ser Ser Met Ser Pro Leu Pro
            420                 425                 430

Gln Met Pro Met Gln Thr Leu Gln Asp Ser Lys Ser Ser Tyr Gly Gly
        435                 440                 445

Leu Asn Gln Tyr Asn Cys Ala Pro Gly Leu Leu Lys Glu Leu Leu Thr
    450                 455                 460

Ser Asp Ser Pro Pro His Asn Asp Ile Met Ser Pro Val Asp Pro Gly
465                 470                 475                 480

Val Ala Gln Pro Asn Ser Arg Val Leu Gly Gln Asn Val Met Met Gly
                485                 490                 495

Pro Asn Ser Val Met Pro Ala Tyr Gly Ser Gln Ala Ser His Asn Lys
            500                 505                 510

Met Met Asn Pro Ser Ser His Thr His Pro Gly His Ala Gln Gln Thr
        515                 520                 525

Ala Ser Val Asn Gly Arg Thr Leu Pro His Val Val Asn Thr Met Pro
    530                 535                 540

His Thr Ser Ala Met Asn Arg Leu Thr Pro Val Lys Thr Pro Leu Gln
545                 550                 555                 560

Val Pro Leu Ser His Pro Met Gln Met Ser Ala Leu Gly Ser Tyr Ser
                565                 570                 575

Ser Val Ser Ser Cys Asn Gly Tyr Gly Arg Met Gly Val Leu His Gln
            580                 585                 590

Glu Lys Leu Pro Ser Asp Leu Asp Gly Met Phe Ile Glu Arg Leu Asp
        595                 600                 605

Cys Asp Met Glu Ser Ile Ile Arg Asn Asp Leu Met Asp Gly Asp Thr
    610                 615                 620

Leu Asp Phe Asn Phe Asp Asn Val Leu Pro Asn Gln Ser Phe Pro His
625                 630                 635                 640

Ser Val Lys Thr Thr Thr His Ser Trp Val Ser Gly
                645                 650

<210>  2
<211>  655
<212>  PRT
<213>  Homo sapiens

<400>  2

<210>  3
<211>  5552
<212>  DNA
<213>  Mus musculus

<400>  3
aggggcgggg cggcgcgcgc gccgccgcgg gcggggagcc cgctgcagat cccgtaagac      60

gggagtctgc ggagtcgctt cagtccccgc cgccgccaca ttcaacaggc agcagcgccg     120

ctgtcgcgcg gccgcggaga gctagagcgg cccgcagcgt ccgcccgtct gccttggcgt     180

ccgcggccct tgtcagcggg agcgcggtgc ccgagctgcc gggctccgcg gcctggtcgg     240

tgccccgtcc taggcacgaa ctcggaggct ccttagacac cggtgaccca gcgaagttaa     300

gttctgggcg cgtccgtccg ctgcgccccg ccgcgcctga ctccggcgtg cgtccgccgt     360

ccgcggcccc ccaatctcgg agcgacactc gggtcgcccg ctccgcgccc ccggtggccg     420

cgtctcccgg tacttctctg ctggtggggg aggggcgggg gcaccatggc cgaagcgccc     480

caggtggtgg agaccgaccc ggacttcgag ccgctgcccc ggcagcgctc ctgtacctgg     540

ccgctgccca ggccggagtt taaccagtcc aactcgacca cctccagtcc ggcgccgtcg 600

ggcggcgcgg ccgccaaccc cgacgccgcg gcgagcctgg cctcggcgtc cgctgtcagc 660

accgacttta tgagcaacct gagcctgctg gaggagagtg aggacttcgc gcgggcgcca 720

ggctgcgtgg ccgtggcggc ggcggctgcg gccagcaggg gcctgtgcgg ggacttccag 780

ggccccgagg cgggctgcgt gcacccagcg ccgccacagc ccccaccgac cgggccgctg 840

tcgcagcccc cacccgtgcc tccctccgct gccgccgccg cggggccact cgcgggacag 900

ccgcgcaaga ccagctcgtc gcgccgcaac gcgtggggca acctgtcgta cgccgacctc 960

atcaccaagg ccatcgagag ctcagccgag aagaggctca ccctgtcgca gatctacgag 1020

tggatggtga agagcgtgcc ctacttcaag gataagggcg acagcaacag ctcggcgggc 1080

tggaagaatt caattcgcca caatctgtcc cttcacagca agtttattcg agtgcagaat 1140

gaaggaactg gaaagagttc ttggtggatg ctcaatccag agggaggcaa gagcggaaaa 1200

tcaccccgga gaagagctgc gtccatggac aacaacagta aatttgctaa gagccgaggg 1260

cgggctgcta agaaaaaagc atctctccag tctgggcaag agggtcctgg agacagccct 1320

gggtctcagt tttctaagtg gcctgcgagt cctgggtccc acagcaacga tgactttgat 1380

aactggagta catttcgtcc tcgaaccagc tcaaatgcta gtaccatcag tgggagactt 1440

tctcccatca tgacagagca ggatgacctg ggagatgggg acgtgcattc cctggtgtat 1500

ccaccctctg ctgccaagat ggcgtctacg ctgcccagtc tgtctgaaat cagcaatcca 1560

gaaaacatgg agaaccttct ggataatctc aaccttctct cgtccccaac atctttaact 1620

gtgtccaccc agtcctcgcc tggcagcatg atgcagcaga caccatgcta ttcgtttgca 1680

ccgccaaaca ccagtctaaa ttcacccagt ccaaactact caaagtacac atacggccaa 1740

tccagcatga gccctttgcc ccagatgcct atgcagacac ttcaggacag caaatcaagt 1800

tacggaggat tgaaccagta taactgtgcc ccaggactct tgaaagagtt gttgacttct 1860

gactctcctc cccacaatga cattatgtca ccggttgatc ccggagtggc ccaacccaac 1920

agtcgggtcc tgggccaaaa tgtaatgatg ggccctaatt cggtcatgcc agcgtatggc 1980

agccaggcat ctcataacaa aatgatgaac cccagctccc acacccaccc tggacatgca 2040

cagcaaacgg cttcggtcaa cggccgtacc ctgccccatg tggtgaacac catgcctcac 2100

acatctgcca tgaaccgctt gacccccgtg aagacacctt tacaagtgcc tctgtcccac 2160

cccatgcaga tgagtgccct gggcagctac tcctcggtga gcagctgcaa tggctatggt 2220

aggatgggtg tcctccacca ggagaagctc ccaagtgact tggatggcat gtttattgag 2280

cgcttggact gtgacatgga gtccatcatt cggaatgacc tcatggatgg agataccttg 2340

gattttaact ttgataatgt gttgcccaac caaagcttcc cacacagtgt caagactaca 2400

acacacagct gggtgtcagg ctaagagtta gtgagcaggc tacatttaaa agtccttcag 2460

attgtctgac agcaggaact gaggagcagt ccaaagatgc ccttcacccc tccttatagt 2520

tttcaagatt taaaaaaaaa aaaaaaaaaa aaaaaagtcc tttctccttt cctcagactt 2580

ggcaacagcg gcagcacttt cctgtgcagg atgtttgccc agcgtccgca ggttttgtgc 2640

tcctgtagat aaggactgtg ccattgggaa tcattacaat gaagtgccaa actcactaca 2700

ccatgtaatt gcagaaaaga ctttcagatc ctggagtgct ttcaagtttt gtatatatgc 2760

agtagataca gaattgtatt tgtgtgtgtg ttttttaata cctacttggt ccaaggaaag 2820

tttatactct tttgtaatac tgtgatggtc tcaagtcttg ataaactttg ctttgtacta 2880

cctgtgttct gctacagtga gaagtcatga actaagatct ctgtcctgca cctcggctga 2940

atgactgaac ctggtcattt gccacagaac ccatgagagc caagtagcca gtgatcaatg 3000

tgctgaatta atggacttgt caaactttgg ggcagaataa gattaagtgc cagctttgta 3060

caggtctttt tctattgttt ttgttgttgt ttattttgtt atttgcaaat ttgtacaaac 3120

aacttaaaat ggttctaatt tccagataaa tgacttttga tgttattgtt aggactcaac 3180

atcttttgga atagataccg aagtgtaatg ttttcttaaa actagagtct actttgttac 3240

attgtctgct tataaatttg tgaaatcaga ggtatttggg ggctgcattc ataattttca 3300

ttttgtattt ctaactggat tagtactaat tttatatgtg ctcagctggt ttgtacactt 3360

tgcgatgata cctgataatg tttctgacta atcgtaaacc attgtaatta gtacttgcac 3420

actcaacgtt cctggccctt tgggcaggaa agttatgtat agttacagac actctgtttt 3480

gtgtgtagat ttatgtgtgt attttaaaga aatttcacct gcttttatta ccctgtgagt 3540

tgtgtacagc gcatagcacc aagtcttcag atagatgcca cgtgcttaca gccttctagg 3600

gaagcctgcc agatgatgcc ctgtgtcacg ctgtcatagt tcccatggga actctgtctg 3660

tcgctcagga aaggggaact tttatctaag gtgatgttct ttgtctgact ggggttcgcc 3720

tcctactact ctgagctgtt ggcttttgtc acgatggagg tggctttgtg gctctgtcct 3780

gaagaatcct gtcacttctc ggtccccacc tctgttctct ttggctctga acagtgtaaa 3840

tctaaggagg aagtttacaa ataggacttc agtgatttat ggagtgctct gtgcgcctaa 3900

gtacagacag tggcaggatt agttaaaaat gaaggcagta aacttggaaa ccagccagct 3960

ataaatggac atttattttg aaatccttag cttaagaatt tgagaagttt tttcagcctt 4020

gagcagccta atgtgtctca aacatttacg ttttttatac attctattta cctgaaatcc 4080

tgccagacca ggataattgg ttttacctct cattccgtcc atcggtgttt cccagtctcc 4140

cacagtttga ggaatagatg taccccagca cccctctttg cctttatgag aaggcctggt 4200

ttgcatgaga agaccaaatt gcacttccat gagaagacca aattgtttgt agtgttactt 4260

agctctcccc tcgtttgtta gtgtgtgtta acaagaataa aatgtccctg ctttcaccca 4320

ccgttggcca gctttgtcat aggcttccca ccataacttt cactatttta aacacatatt 4380

gagccactgc tcgtctgact acctttgttt gggcactcca aaacaggact tgttttagaa 4440

atgaactcct ccaagtagag cctccttcaa acagagtaga atttcctggt gtcaaagaac 4500

ccgggtctgt ctccctttcc tcctccctct gccatttctt accattgcgg aaagagagag 4560

cctccgtgtg taatcattca gtagaggcag ctaccgccct ggcagtggtc tacctgctga 4620

atgccactga atgactagga ggtgtctctc ccttcagaag ctgtcaattt cagcagcaac 4680

ccctgttttc cttggtgtta agatcccagt gtgaatcatg ggcagttgtc tggggcacag 4740

tgaactccag gaaaggcttc gtatctgttt tgaaaacaaa catcaaacgt gtgagctccg 4800

agggtccttt tctgggagaa tgttcgcttt ctggtctatt attgtacatg attgctctgt 4860

gaaaagactt catctatgca gccttgtttg attcatttcc tttggtgtgt tctgttgtta 4920

agagcaaatt gtattataga gctatttgga tattttaaat ataaagatgt attgtttcca 4980

taatatagat gtatggagta tatttaggtg atagatgtac aacttggaaa gttctgcttg 5040

gacaaactga gtctaagtta attagcaaat aatatatcct gatgagcagg aagccctgaa 5100

acctaacaac agtaagcgga gaaaatcact taaaatggaa acagttcccc aaaggtgttc 5160

aatttgaact tgttcaactg cttaatatat ggtccccccc ccccccaaaa aaaaaacctt 5220

gaagttctta gttttcagct ctccaagtta ctgattttaa gtgaagtttc tctgtggttt 5280

cagctgggga gtgattgttc agtagagtgt gcattgtgct ttatgcaaac caaacagcct 5340

ggccctgtgg ccggggacag acagacagcc cgtcaggata gagtcccgcc cttcgccacc 5400

acagcggact tgagtaacag tgcagatgcc ttgctcctgt tccattgcta tctgagaagt 5460

gcctgatgag gatggtaaac ttacagacac aagaacaatc cttactgtgc gttgtataaa 5520

gccataaatg tacataaatc atcttaagtg gc 5552

<210> 4
<211> 5738
<212> DNA
<213> Homo sapiens

<400> 4
gcagccgcca cattcaacag gcagcagcgc agcgggcgcg ccgctgggga gagcaagcgg 60

cccgcggcgt ccgtccgtcc ttccgtccgc ggccctgtca gctggagcgc ggcgcaggct 120

ctgccccggc ccggcggctc tggccggccg tccagtccgt gcggcggacc ccgaggagcc 180

tcgatgtgga tggccccgcg aagttaagtt ctgggctcgc gcttccactc cgccgcgcct 240

tcctcccagt ttccgtccgc tcgccgcacc ggcttcgttc ccccaaatct cggaccgtcc 300

cttcgcgccc cctccccgtc cgcccccagt gctgcgttct ccccctcttg gctctcctgc 360

ggctggggga ggggcggggg tcaccatggc cgaggcgcct caggtggtgg agatcgaccc 420

ggacttcgag ccgctgcccc ggccgcgctc gtgcacctgg ccgctgccca ggccggagtt 480
tagccagtcc aactcggcca cctccagccc ggcgccgtcg ggcagcgcgg ctgccaaccc 540
cgacgccgcg gcgggcctgc cctcggcctc ggctgccgct gtcagcgccg acttcatgag 600
caacctgagc ttgctggagg agagcgagga cttcccgcag gcgcccggct ccgtggcggc 660
ggcggtggcg gcggcggccg ccgcggccgc caccgggggg ctgtgcgggg acttccaggg 720
cccggaggcg ggctgcctgc acccagcgcc accgcagccc ccgccgcccg ggccgctgtc 780
gcagcacccg ccggtgcccc ccgccgccgc tgggccgctc gcggggcagc cgcgcaagag 840
cagctcgtcc cgccgcaacg cgtggggcaa cctgtcctac gccgacctca tcaccaaggc 900
catcgagagc tcggcggaga agcggctcac gctgtcgcag atctacgagt ggatggtcaa 960
gagcgtgccc tacttcaagg ataagggtga cagcaacagc tcggcgggct ggaagaattc 1020
aattcgtcat aatctgtccc tacacagcaa gttcattcgt gtgcagaatg aaggaactgg 1080
aaaaagttct tggtggatgc tcaatccaga gggtggcaag agcgggaaat ctcctaggag 1140
aagagctgca tccatggaca acaacagtaa atttgctaag agccgaagcc gagctgccaa 1200
gaagaaagca tctctccagt ctggccagga gggtgctggg gacagccctg gatcacagtt 1260
ttccaaatgg cctgcaagcc ctggctctca cagcaatgat gactttgata actggagtac 1320
atttcgccct cgaactagct caaatgctag tactattagt gggagactct cacccattat 1380
gaccgaacag gatgatcttg gagaagggga tgtgcattct atggtgtacc cgccatctgc 1440
cgcaaagatg gcctctactt tacccagtct gtctgagata agcaatcccg aaaacatgga 1500
aaatcttttg gataatctca accttctctc atcaccaaca tcattaactg tttcgaccca 1560
gtcctcacct ggcaccatga tgcagcagac gccgtgctac tcgtttgcgc caccaaacac 1620
cagtttgaat tcacccagcc caaactacca aaaatataca tatggccaat ccagcatgag 1680
ccctttgccc cagatgccta tacaaacact tcaggacaat aagtcgagtt atggaggtat 1740
gagtcagtat aactgtgcgc ctggactctt gaaggagttg ctgacttctg actctcctcc 1800
ccataatgac attatgacac cagttgatcc tggggtagcc cagcccaaca gccgggttct 1860
gggccagaac gtcatgatgg gccctaattc ggtcatgtca acctatggca gccaggcatc 1920
tcataacaaa atgatgaatc ccagctccca tacccaccct ggacatgctc agcagacatc 1980
tgcagttaac gggcgtcccc tgccccacac ggtaagcacc atgccccaca cctcgggtat 2040
gaaccgcctg acccaagtga agacacctgt acaagtgcct ctgccccacc ccatgcagat 2100
gagtgccctg gggggctact cctccgtgag cagctgcaat ggctatggca gaatgggcct 2160
tctccaccag gagaagctcc caagtgactt ggatggcatg ttcattgagc gcttagactg 2220
tgacatggaa tccatcattc ggaatgacct catggatgga gatacattgg attttaactt 2280
tgacaatgtg ttgcccaacc aaagcttccc acacagtgtc aagacaacga cacatagctg 2340

ggtgtcaggc tgagggttag tgagcaggtt acacttaaaa gtacttcaga ttgtctgaca 2400

gcaggaactg agagaagcag tccaaagatg tctttcacca actccctttt agttttcttg 2460

gttaaaaaaa aaaacaaaaa aaaaaaccct ccttttttcc tttcgtcaga cttggcagca 2520

aagacatttt tcctgtacag gatgtttgcc caatgtgtgc aggttatgtg ctgctgtaga 2580

taaggactgt gccattggaa atttcattac aatgaagtgc caaactcact acaccatata 2640

attgcagaaa agattttcag atcctggtgt gctttcaagt tttgtatata agcagtagat 2700

acagattgta tttgtgtgtg tttttggttt ttctaaatat ccaattggtc caaggaaagt 2760

ttatactctt tttgtaatac tgtgatgggc ctcatgtctt gataagttaa actttgtttt 2820

gtactacctg ttttctgcgg aactgacgga tcacaaagaa ctgaatctcc attctgcatc 2880

tccattgaac agccttggac ctgttcacgt tgccacagaa ttcacatgag aaccaagtag 2940

cctgttatca atctgctaaa ttaatggact tgttaaactt ttggaaaaaa aaagattaaa 3000

tgccagcttt gtacaggtct tttctatttt tttttgttta ttttgttatt tgcaaatttg 3060

tacaaacatt taaatggttc taatttccag ataaatgatt tttgatgtta ttgttgggac 3120

ttaagaacat ttttggaata gatattgaac tgtaataatg ttttcttaaa actagagtct 3180

actttgttac atagtcagct tgtaaatttt gtggaaccac aggtatttgg ggcagcattc 3240

ataattttca ttttgtattc taactggatt agtactaatt ttatacatgc ttaactggtt 3300

tgtacacttt gggatgctac ttagtgatgt ttctgactaa tcttaaatca ttgtaattag 3360

tacttgcata ttcaacgttt caggccctgg ttgggcagga aagtgatgta tagttatgga 3420

cactttgcgt ttcttattta ggataactta atatgttttt atgtatgtat tttaaagaaa 3480

tttcatctgc ttctactgaa ctatgcgtac tgcatagcat caagtcttct ctagagacct 3540

ctgtagtcct gggaggcctc ataatgtttg tagatcagaa aagggagatc tgcatctaaa 3600

gcaatggtcc tttgtcaaac gagggatttt gatccacttc accattttga gttgagcttt 3660

agcaaaagtt tcccctcata attctttgct cttgtttcag tccaggtgga ggttggtttt 3720

gtagttctgc cttgaggaat tatgtcaaca ctcatacttc atctcattct cccttctgcc 3780

ctgcagatta gattacttag cacactgtgg aagtttaagt ggaaggaggg aatttaaaaa 3840

tgggacttga gtggtttgta gaatttgtgt tcataagttc agatgggtag caaatggaat 3900

agaacttact taaaaattgg ggagatttat ttgaaaacca gctgtaagtt gtgcattgag 3960

attatgttaa aagccttggc ttaagaattt gaaaatttct ttagcctgta gcaacctaaa 4020

ctgtaattcc tatcattatg ttttattact ttccaattac ctgtaactga cagaccaaat 4080

taattggctt tgtgtcctat ttagtccatc agtattttca agtcatgtgg aaagcccaaa 4140

gtcatcacaa tgaagagaac aggtgcacag cactgttcct cttgtgttct tgagaaggat 4200

ctaatttttc tgtatatagc ccacatcaca cttgctttgt cttgtatgtt aattgcatct 4260

tcattggctt ggtatttcct aaatgtttaa caagaacaca agtgttcctg ataagatttc 4320

ctacagtaag ccagctctat tgtaagcttc ccactgtgat gatcattttt ttgaagattc 4380

attgaacagc caccactcta tcatcctcat tttggggcag tccaagacat agctggtttt 4440

agaaacccaa gttcctctaa gcacagcctc ccgggtatgt aactgaactt ggtgccaaag 4500

tacttgtgta ctaatttcta ttactacgta ctgtcacttt cctcccgtgc cattactgca 4560

tcataataca aggaacctca gagcccccat ttgttcatta aagaggcaac tacagccaaa 4620

atcactgtta aaatcttact acttcatgga gtagctctta ggaaaatata tcttcctcct 4680

gagtctgggt aattatacct ctcccaagcc cccattgtgt gttgaaatcc tgtcatgaat 4740

ccttggtagc tctctgagaa cagtgaagtc cagggaaagg catctggtct gtctggaaag 4800

caaacattat gtggcctctg gtagtttttt tcctgtaaga atactgactt tctggagtaa 4860

tgagtatata tcagttattg tacatgattg ctttgtgaaa tgtgcaaatg atatcaccta 4920

tgcagccttg tttgatttat tttctctggt ttgtactgtt attaaaagca tattgtatta 4980

tagagctatt cagatatttt aaatataaag atgtattgtt tccgtaatat agacgtatgg 5040

aatatattta ggtaatagat gtattacttg gaaagttctg ctttgacaaa ctgacaaagt 5100

ctaaatgagc acatgtatcc cagtgagcag taaatcaatg gaacatccca agaagaggat 5160

aaggatgctt aaaatggaaa tcattctcca acgatataca aattggactt gttcaactgc 5220

tggatatatg ctaccaataa ccccagcccc aacttaaaat tcttacattc aagctcctaa 5280

gagttcttaa tttataacta attttaaaag agaagtttct tttctggttt tagtttggga 5340

ataatcattc attaaaaaaa atgtattgtg gtttatgcga acagaccaac ctggcattac 5400

agttggcctc tccttgaggt gggcacagcc tggcagtgtg gccaggggtg gccatgtaag 5460

tcccatcagg acgtagtcat gcctcctgca tttcgctacc cgagtttagt aacagtgcag 5520

attccacgtt cttgttccga tactctgaga agtgcctgat gttgatgtac ttacagacac 5580

aagaacaatc tttgctataa ttgtataaag ccataaatgt acataaatta tgtttaaatg 5640

gcttggtgtc tttcttttct aattatgcag aataagctct ttattaggaa ttttttgtga 5700

agctattaaa tacttgagtt aagtcttgtc agccacaa 5738

<210> 5
<211> 20
<212> DNA
<213> Artificial Sequence

<220>
<223> forward primer

<400> 5
aaggccaacc gtgaaaagat 20

<210>  6
<211>  21
<212>  DNA
<213>  Artificial Sequence

<220>
<223>  reverse primer

<400>  6
gtggtacgac cagagggata c                                                21


<210>  7
<211>  21
<212>  DNA
<213>  Artificial Sequence

<220>
<223>  forward primer

<400>  7
cagattgatg gctctttctc g                                                21


<210>  8
<211>  20
<212>  DNA
<213>  Artificial Sequence

<220>
<223>  reverse primer

<400>  8
agacaaatcg ctccaccaac                                                  20


<210>  9
<211>  20
<212>  DNA
<213>  Artificial Sequence

<220>
<223>  forward primer

<400>  9
aggtagctgt caacaaggca                                                  20


<210>  10
<211>  20
<212>  DNA
<213>  Artificial Sequence

<220>
<223>  reverse primer

<400>  10
cttgccgagg agtttgagat                                                  20


<210>  11

<211>  20
<212>  DNA
<213>  Artificial Sequence

<220>
<223>  forward primer

<400>  11
cgaagtgtgg tatcctggtg                                                   20


<210>  12
<211>  20
<212>  DNA
<213>  Artificial Sequence

<220>
<223>  reverse primer

<400>  12
ggtactgtcc aaacgcatgt                                                   20


<210>  13
<211>  20
<212>  DNA
<213>  Artificial Sequence

<220>
<223>  forward primer

<400>  13
ttttctagga gcagcggtgt                                                   20


<210>  14
<211>  20
<212>  DNA
<213>  Artificial Sequence

<220>
<223>  reverse primer

<400>  14
agtggaaaag agcctgccaa                                                   20


<210>  15
<211>  20
<212>  DNA
<213>  Artificial Sequence

<220>
<223>  forward primer

<400>  15
catcgaggtg agtgtcaagg                                                   20


<210>  16
<211>  20
<212>  DNA
<213>  Artificial Sequence

<220>
<223>  reverse primer

<400>  16
cagacacgca ccacttcttt                                                    20


<210>  17
<211>  20
<212>  DNA
<213>  Artificial Sequence

<220>
<223>  forward primer

<400>  17
ggactagcaa gagcctttgg                                                    20


<210>  18
<211>  20
<212>  DNA
<213>  Artificial Sequence

<220>
<223>  reverse primer

<400>  18
aagaatttca ggtgctcggt                                                    20


<210>  19
<211>  20
<212>  DNA
<213>  Artificial Sequence

<220>
<223>  forward primer

<400>  19
agtctcatgg tgtggtggaa                                                    20


<210>  20
<211>  20
<212>  DNA
<213>  Artificial Sequence

<220>
<223>  reverse primer

<400>  20
gacatcacca ggattggaca                                                    20


<210>  21
<211>  20
<212>  DNA
<213>  Artificial Sequence

<220>
<223>  forward primer

<400>  21
agtgtccagg gatgaggaag                                                   20


<210>  22
<211>  20
<212>  DNA
<213>  Artificial Sequence

<220>
<223>  reverse primer

<400>  22
cttctgttct gttggccctt                                                   20


<210>  23
<211>  18
<212>  DNA
<213>  Artificial Sequence

<220>
<223>  forward primer

<400>  23
aatcagccag ccttcgac                                                     18


<210>  24
<211>  20
<212>  DNA
<213>  Artificial Sequence

<220>
<223>  reverse primer

<400>  24
atcactggga aggtatcgct                                                   20


<210>  25
<211>  20
<212>  DNA
<213>  Artificial Sequence

<220>
<223>  forward primer

<400>  25
tccatgaacc agaacctcac                                                   20


<210>  26
<211>  20
<212>  DNA
<213>  Artificial Sequence

<220>
<223>  reverse primer

<400>  26
ggcttcattt ctttggcttc                                                   20

<210>  27
<211>  20
<212>  DNA
<213>  Artificial Sequence

<220>
<223>  forward primer

<400>  27
gcctcaatga tgggcttatt                                                   20


<210>  28
<211>  20
<212>  DNA
<213>  Artificial Sequence

<220>
<223>  reverse primer

<400>  28
aaagcctggc actctctttg                                                   20


<210>  29
<211>  20
<212>  DNA
<213>  Artificial Sequence

<220>
<223>  forward primer

<400>  29
gagaagaggc tcaccctgtc                                                   20


<210>  30
<211>  20
<212>  DNA
<213>  Artificial Sequence

<220>
<223>  reverse primer

<400>  30
acagattgtg gcgaattgaa                                                   20


<210>  31
<211>  21
<212>  DNA
<213>  Artificial Sequence

<220>
<223>  forward primer

<400>  31
tccatgacaa ctttggcatt g                                                 21


<210>  32

<211>  21
<212>  DNA
<213>  Artificial Sequence

<220>
<223>  reverse primer

<400>  32
cagtcttctg ggtggcagtg a                                                 21


<210>  33
<211>  22
<212>  DNA
<213>  Artificial Sequence

<220>
<223>  forward primer

<400>  33
gaggagaacc ccagatcatt cc                                                22


<210>  34
<211>  21
<212>  DNA
<213>  Artificial Sequence

<220>
<223>  reverse primer

<400>  34
tgtgagtggc gtttgtcttc a                                                 21


<210>  35
<211>  17
<212>  DNA
<213>  Artificial Sequence

<220>
<223>  forward primer

<400>  35
ctcgtggcgc tgatgct                                                      17


<210>  36
<211>  28
<212>  DNA
<213>  Artificial Sequence

<220>
<223>  reverse primer

<400>  36
ctggttgaat agtaaaatat cccattga                                          28


<210>  37
<211>  19
<212>  DNA
<213>  Artificial Sequence

<220>
<223>  forward primer

<400>  37
tcaacatggc cctgtggat                                                    19


<210>  38
<211>  21
<212>  DNA
<213>  Artificial Sequence

<220>
<223>  reverse primer

<400>  38
aaaggtgctg cttgaaaaag c                                                 21


<210>  39
<211>  21
<212>  DNA
<213>  Artificial Sequence

<220>
<223>  forward primer

<400>  39
ggaggtcatc cgactgaaac a                                                 21


<210>  40
<211>  21
<212>  DNA
<213>  Artificial Sequence

<220>
<223>  reverse primer

<400>  40
gcacctctcg ctctccagaa t                                                 21


<210>  41
<211>  20
<212>  DNA
<213>  Artificial Sequence

<220>
<223>  forward primer

<400>  41
tgagctagag ggaggaagga                                                   20


<210>  42
<211>  20
<212>  DNA
<213>  Artificial Sequence

<220>
<223>  reverse primer

<400>  42
ccgggtttct ctaactctgc                                                   20


<210>  43
<211>  20
<212>  DNA
<213>  Artificial Sequence

<220>
<223>  forward primer

<400>  43
gtcgggagaa ctaggatggc                                                   20


<210>  44
<211>  19
<212>  DNA
<213>  Artificial Sequence

<220>
<223>  reverse primer

<400>  44
ggagcagtcc ctaggtatg                                                    19


<210>  45
<211>  22
<212>  DNA
<213>  Artificial Sequence

<220>
<223>  forward primer

<400>  45
agagagcacg cttggcctat tc                                                22


<210>  46
<211>  21
<212>  DNA
<213>  Artificial Sequence

<220>
<223>  reverse primer

<400>  46
gtcgtcagag ttcgggtcca g                                                 21


<210>  47
<211>  20
<212>  DNA
<213>  Artificial Sequence

<220>
<223>  forward primer

<400>  47
ggacaaggct cccagtgtgt                                                   20

<210>  48
<211>  22
<212>  DNA
<213>  Artificial Sequence

<220>
<223>  reverse primer

<400>  48
gcaagctctg gtcttccttg aa                                                22


<210>  49
<211>  25
<212>  DNA
<213>  Artificial Sequence

<220>
<223>  forward primer

<400>  49
tggagttgca tataattcca aagtt                                             25


<210>  50
<211>  21
<212>  DNA
<213>  Artificial Sequence

<220>
<223>  reverse primer

<400>  50
ctagcctcaa tggcatcagt t                                                 21


<210>  51
<211>  19
<212>  DNA
<213>  Artificial Sequence

<220>
<223>  forward primer

<400>  51
gcccgggtgt aggcagtac                                                    19


<210>  52
<211>  20
<212>  DNA
<213>  Artificial Sequence

<220>
<223>  reverse primer

<400>  52
cagtgggcag gaggtgctta                                                   20


<210>  53

<211>  22
<212>  DNA
<213>  Artificial Sequence

<220>
<223>  forward primer

<400>  53
taaaagttca gacctttggg cc                                                22


<210>  54
<211>  18
<212>  DNA
<213>  Artificial Sequence

<220>
<223>  reverse primer

<400>  54
tcccggctct gaatggtg                                                     18


<210>  55
<211>  20
<212>  DNA
<213>  Artificial Sequence

<220>
<223>  forward primer

<400>  55
ctctccccca aaccccatat                                                   20


<210>  56
<211>  24
<212>  DNA
<213>  Artificial Sequence

<220>
<223>  reverse primer

<400>  56
tttctaatgc agggtcaagt tgag                                              24


<210>  57
<211>  20
<212>  DNA
<213>  Artificial Sequence

<220>
<223>  forward primer

<400>  57
gaaagcttca gctctttccg                                                   20


<210>  58
<211>  20
<212>  DNA
<213>  Artificial Sequence

<220>
<223>  reverse primer

<400>  58
aggccttcca ggcttagatt                                                   20


<210>  59
<211>  20
<212>  DNA
<213>  Artificial Sequence

<220>
<223>  forward primer

<400>  59
gcacagccac catgaattac                                                   20


<210>  60
<211>  20
<212>  DNA
<213>  Artificial Sequence

<220>
<223>  reverse primer

<400>  60
ggaggtagaa ctggcgtctc                                                   20

## Description

### FIELD OF THE INVENTION

The present invention relates to treatment of diabetes, and more particularly to compositions and methods for converting non-insulin producing cells into insulin producing cells.

### BACKGROUND OF THE INVENTION

In 2012, it was estimated that diabetes was affecting about 347 million people worldwide and this number is still increasing (World Health Organization's data). Diabetes mellitus occurs throughout the world, but is more prevalent (especially type 2) in the more developed countries. The greatest future increase in prevalence is, however, expected to occur in Asia and Africa, where the majority of sufferers will probably be located by 2030.

Diabetes is a chronic disease that occurs either when the pancreas does not produce enough insulin or when the body cannot effectively use the insulin it does produce. Insulin is a hormone that regulates blood sugar. Hyperglycaemia, or raised blood sugar, is a common effect of uncontrolled diabetes and over time leads to serious damage to many of the body's systems, especially the nerves and blood vessels. Underlying defects lead to a classification of diabetes into two major groups: type 1 and type 2. Type 1 diabetes, or insulin dependent diabetes mellitus (IDDM), arises when patients lack insulin-producing β-cells in their pancreatic glands. Type 2 diabetes, or non-insulin dependent diabetes mellitus (NIDDM), occurs in patients with impaired β-cell function and alterations in insulin action.

The current treatment for type 1 diabetic patients is the regular injection of insulin, indicated by frequent glucose monitoring, while the majority of type 2 diabetic patients are treated with agents that stimulate β-cell function or with agents that enhance the tissue sensitivity of the patients towards insulin. The drugs presently used to treat type 2 diabetes include alpha-glucosidase inhibitors, insulin sensitizers, insulin secretagogues, metformin and insulin itself. An alternative therapeutic approach for treating diabetes would consist of cell replacement-based therapy. However, this method is facing the difficulty of supplying or generating vast numbers of compatible functioning insulin-producing β-cells. One way to increase the number of insulin producing cells could be through the reprogramming of alternative endogenous cell types within individual patients. Recent studies reveal significant plasticity of pancreatic α-cells under certain induced conditions, implying a potential route to insulin production by transformed α cells. In a near-total β-cell destruction and regeneration model in adult mice, a proportion of new insulin producing cells were produced from α cells via a bihormonal glucagon⁺ insulin⁺ transitional state (Thorel et al, 2010, Nature 464:1149-1154). Forkhead transcription factors of the FoxO family have important roles in cellular proliferation, apoptosis, differentiation and stress resistance (Kitamura et al, 2011, Islet Cell Growth Factors, Edited by Rohit N Kulkarni, Landes Bioscience, chapter 6*;* Accili and Arden, 2004, Cell 117, 421-426*).* Forkhead box protein O1 ("FoxO1" in mice, "FOXO1" in humans) regulates glucose and lipid metabolism in liver, as well as preadipocyte, myoblast and vascular endothelial cell differentiation. The expression pattern of FoxO1 during pancreatic organogenesis is similar to that of Pdx1, Nkx2.2 and Pax4, transcription factors known to be critical for β-cell development. A series of studies on FoxO1 in pancreas suggested that FoxO1 plays important roles in pancreatic β-cell differentiation, neogenesis, proliferation and stress resistance. The contribution of FOXO 1 signalling to the development of β-cell failure in Type II diabetes has also been postulated *(*Kitamura, 2013, Nat Rev Endocrinol. 9(10):615-623*).*

Synthetic, optimized antisense oligonucleotides (ASOs) specifically inhibit FoxO1 expression. In mice with diet-induced obesity (DIO) FoxO1 ASO therapy improved both hepatic insulin and peripheral insulin action (Samuel et al., 2006, Diabetes 55, 2042-2050*).* In a similar manner, functional inhibition of FoxO1, caused by hepatic expression of its mutant, is associated with reduced hepatic gluconeogenic activity and improved fasting glycemia in diabetic mice (Altomonte et al., 2003, Am. J. Physiol. Endocrinol. Metab. 285, E718-E728*)*

In another study, somatic functional FoxO1 ablation in gut epithelium gives rise to gut insulin-positive cells that express markers characteristic of mature β cells *(*Talchai et al., 2012, Nat Genet. 44(4):406-12*).*

Haploinsufficiency of the FoxO1 gene restores insulin sensitivity and rescues the diabetic phenotype in insulin-resistant mice. On the other hand, a gain-of-function FoxO1 mutation targeted to liver and pancreatic beta-cells results in diabetes (Nakae et al., 2002, Nat. Genet. 32(2):245-53*).*

Methods to modulate FOXO1 subcellular localisation have been described in US 2009/0156523.

It has been shown that oral administration of one inhibitor of FoxO1 (AS 1842856) to diabetic db/db mice led to a drastic decrease in fasting plasma glucose level via inhibition of hepatic gluconeogenic genes, suggesting its use for treating type 2 diabetes via a direct effect upon glucose metabolism. *(*Nagashima et al, 2010, Molecular pharmacology 78:961-970*).*

Despite progress in therapy and patient management through lifestyle, diet and drug treatment, a great need still exists for compositions and methods for the successful treatment and management of diabetes. A better understanding of the potential to exploit plasticity between cells and the agents that may facilitate such plasticity, would result in new therapeutic strategies with enhanced treatment potential and improved quality of life for sufferers.

### SUMMARY OF THE INVENTION

The inventors have surprisingly observed a previously undescribed spontaneous regeneration pathway in juvenile mice after near total β-cell destruction, involving the de-differentiation of pancreatic δ-cells and their subsequent re-differentiation to functional insulin-producing cells. Furthermore, it has been surprisingly noted that such spontaneous juvenile regeneration may be artificially stimulated in mice by modulation of certain factors, notably Forkhead transcription factors.

Thus, a first aspect of the invention provides a method of inducing insulin production in δ-cells comprising the step of inhibiting FOXO1 expression and/or activity in said δ-cells.

A second aspect of the invention relates to a method of converting δ-cells into insulin producing cells comprising the step of inhibiting FOXO1 expression and/or activity in said δ-cells.

A third aspect of the invention relates to a method of preventing and/or treating diabetes comprising the administration of a therapeutically effective amount of at least one FOXO1 inhibitor targeting pancreatic islets or δ-cells in a subject in need thereof.

A fourth aspect of the invention relates to a method of preventing and/or treating diabetes in a subject in need thereof comprising auto-grafting or allo-grafting of δ-cells converted into insulin-producing cells as described herewith.

A fifth aspect of the invention concerns the use of a FOXO1 inhibitor targeting pancreatic islets or δ-cells in the manufacture of a medicament for the treatment and/or prevention of diabetes.

A sixth aspect of the invention is a use of δ-cells converted into insulin producing cells in the manufacture of a medicament for the treatment and/or prevention of diabetes.

A seventh aspect of the invention resides in a FOXO1 inhibitor targeting pancreatic islets or δ-cells for use in preventing and/or treating diabetes.

An eighth aspect of the invention resides in isolated pancreatic islets or isolated δ-cells comprising δ-cells converted into insulin producing cells for use in preventing and/or treating diabetes.

A ninth aspect of the invention concerns a composition in particular a pharmaceutical composition, comprising (i) at least one FOXO1 inhibitor, optionally in a form allowing targeting pancreatic islets or δ-cells and/or (ii) isolated δ-cells converted into insulin producing cells.

A tenth aspect of the invention relates to a method of screening a compound for its ability to inhibit FOXO1 expression and/or activity comprising:
a) Exposing isolated pancreatic islets or isolated δ-cells, comprising δ-cells expressing FOXO1, to a test compound;
b) determining the number of cells which are insulin producing cells in presence and in absence of the test compound;
c) comparing the two values of number of insulin producing cells determined in step b), wherein a number of insulin producing cells that is higher in presence of the test compound compared to the number determined in absence of the test compound is indicative of a test compound able to inhibit FOXO1 expression and/or activity.

An eleventh aspect of the invention relates to FOXO1 inhibitors targeting pancreatic islets or δ-cells.

A twelfth aspect of the invention relates to FOXO1 inhibitors targeting pancreatic islets or δ-cells for use as a medicament.

A thirteenth aspect of the invention resides in isolated δ-cells, optionally within isolated pancreatic islets, converted into insulin producing cells.

A fourteenth aspect of the invention resides in isolated δ-cells, optionally within isolated pancreatic islets, converted into insulin producing cells for use as a medicament.

Other features and advantages of the invention will be apparent from the following detailed description.

### DESCRIPTION OF THE FIGURES

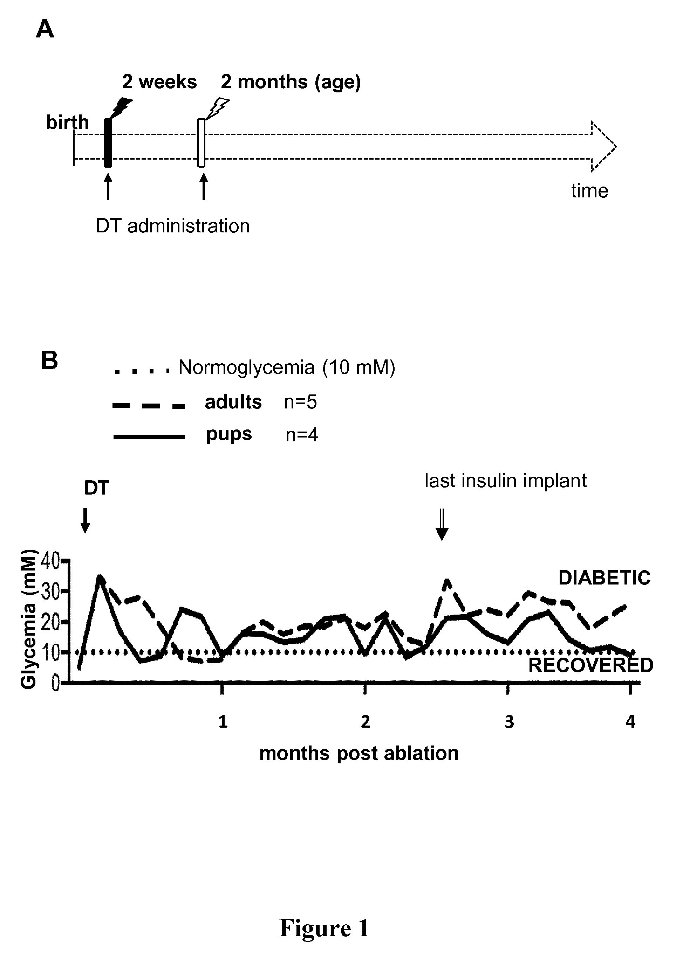

**Figure 1** shows β-cell ablation before puberty, and recovery. **(A)** experimental design depicting the age at DT administration in pups (2-week-old) and post-pubertal (2-month-old mice), **(B)** comparative evolution of glycemia in β-cell ablated pups and middle-aged adults, insulin administration was initiated at β-cell ablation and stopped 2.5 months later.

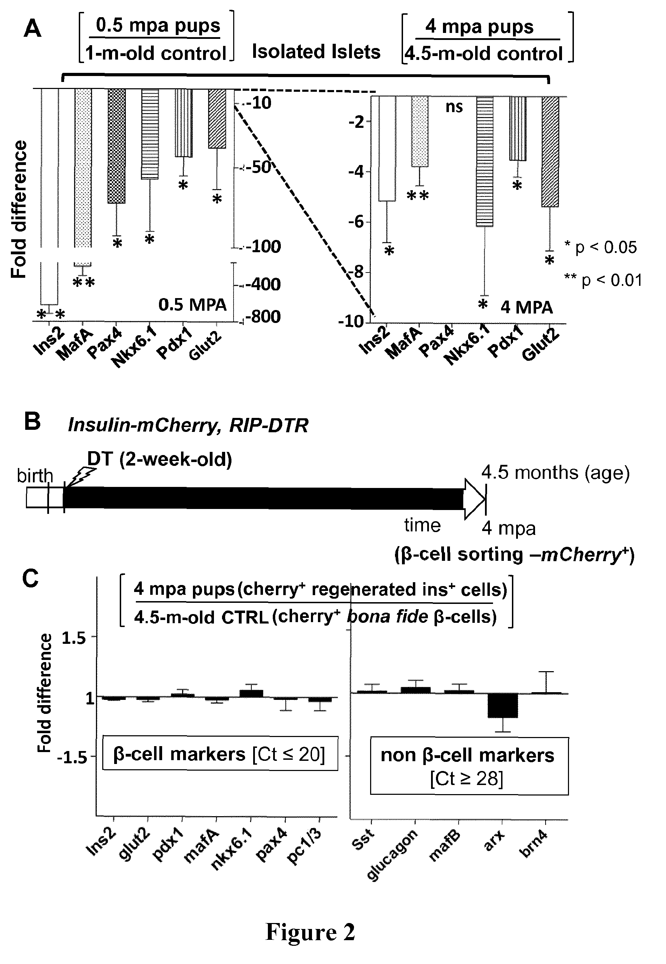

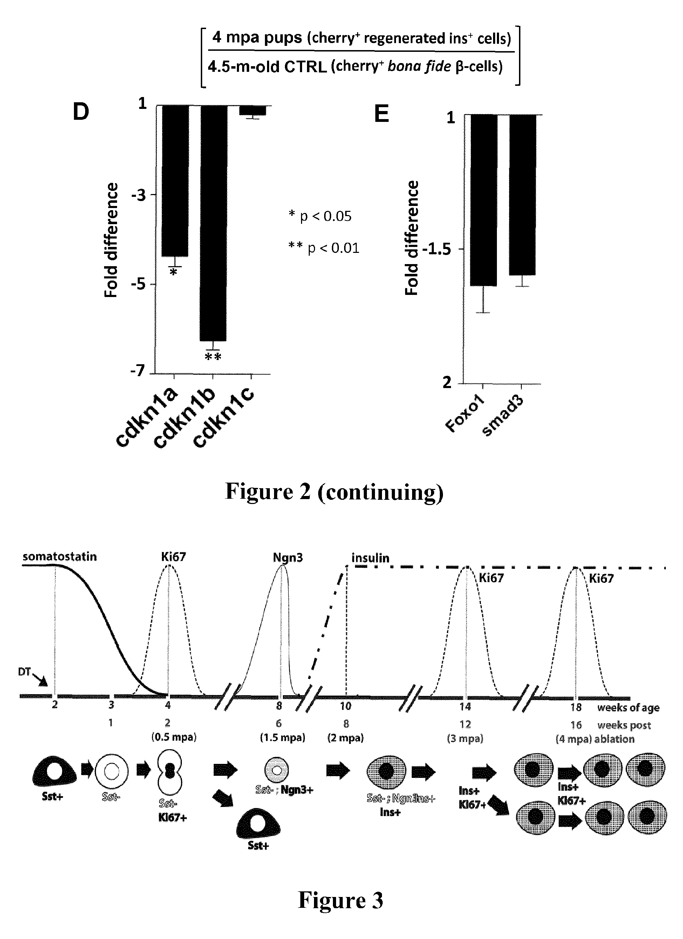

**Figure 2** shows the molecular characterization of δ-cell-derived regenerated insulin⁺ cells. **(A)** qPCR for β-cell-specific genes using RNA extracted from islets isolated from control and DT-treated mice, either 2 weeks or 4 months following DT administration (or months post ablation (mpa): "0.5 mpa" and "4 mpa"). Values represent the ratio between each regeneration time-point and its age-matched control, **(B)** experimental design, **(C)** qPCR comparison between regenerated cherry⁺/insulin⁺ cells isolated from mice 4 months after β-cell ablation, and *bona fide* cherry⁺ β-cells obtained from age-matched controls (4.5-month-old), **(D)** qPCR showing downregulation of cyclin-dependent kinase inhibitors in regenerated cherry⁺/insulin⁺ cells isolated from mice 4 months after β-cell ablation as compared to *bona fide* cherry⁺ β-cells obtained from age-matched controls (4.5-month-old), **(E)** qPCR showing downregulation of FoxO1 and Smad3 in regenerated cherry⁺/insulin⁺ cells isolated from mice 4 months after β-cell ablation as compared to *bona fide* cherry⁺ β-cells obtained from age-matched controls (4.5-month-old).
**Figure 3** shows the sequence of events leading to δ-cell conversion into insulin-producing cells after extreme β-cell loss in juvenile mice: δ-cells dedifferentiate, proliferate and reprogram into insulin production.

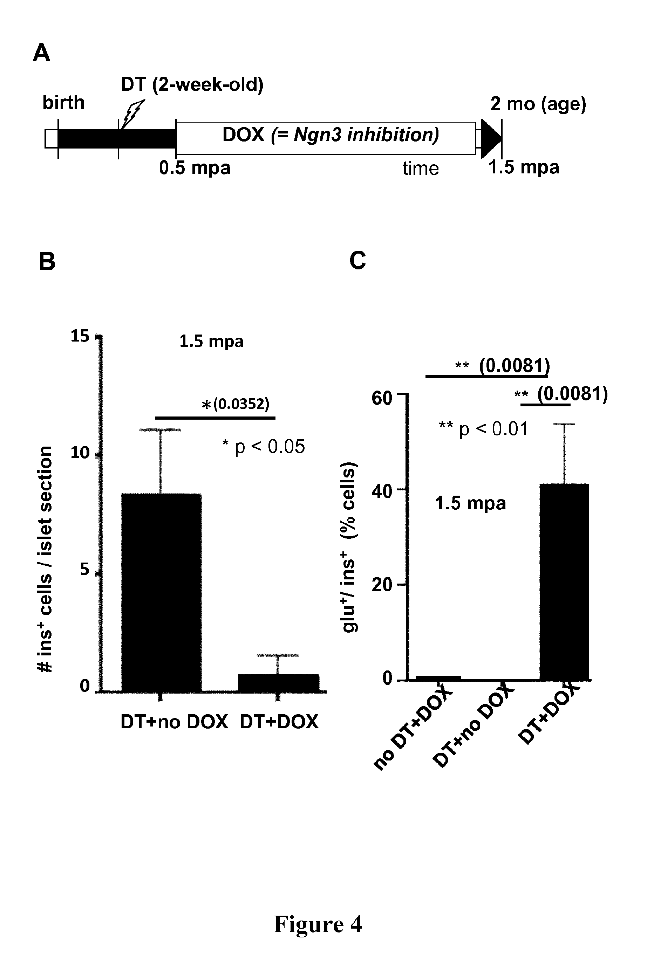

**Figure 4** shows that Ngn3 activation is required for insulin expression in de-differentiated δ-cells. **(A)** Experimental design to block Ngn3 upregulation in β-cell-ablated prepubescent mice, with DOX administration. **(B)** Sharply decreased regeneration of insulin⁺ cells by blocking Ngn3 expression in DOX-treated mice. **(C)** glucagon⁺/insulin⁺ bihormonal cells appear in DOX-treated β-cell-ablated pups (Ngn3 inhibition).

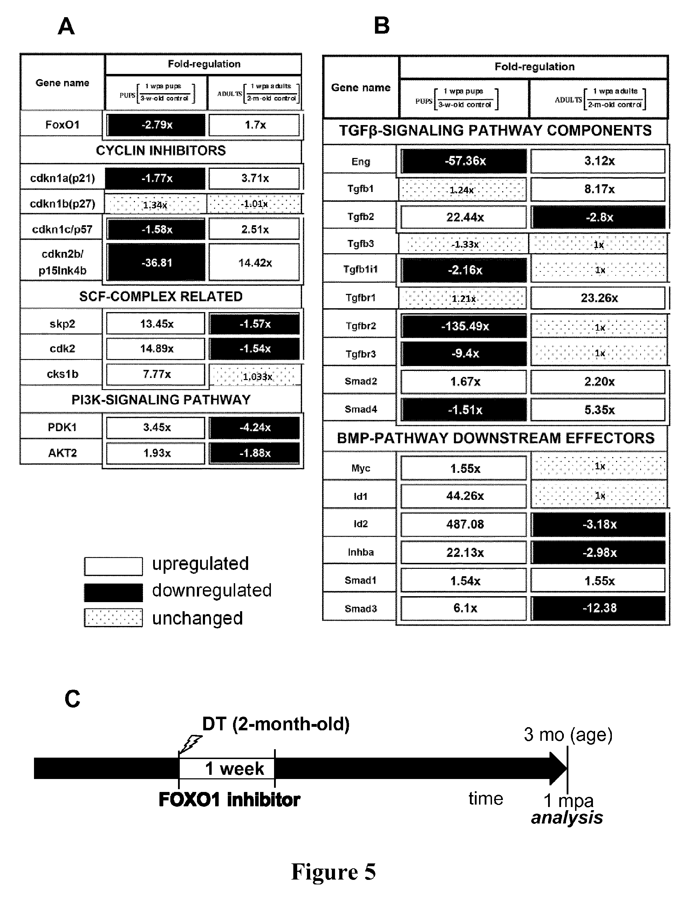

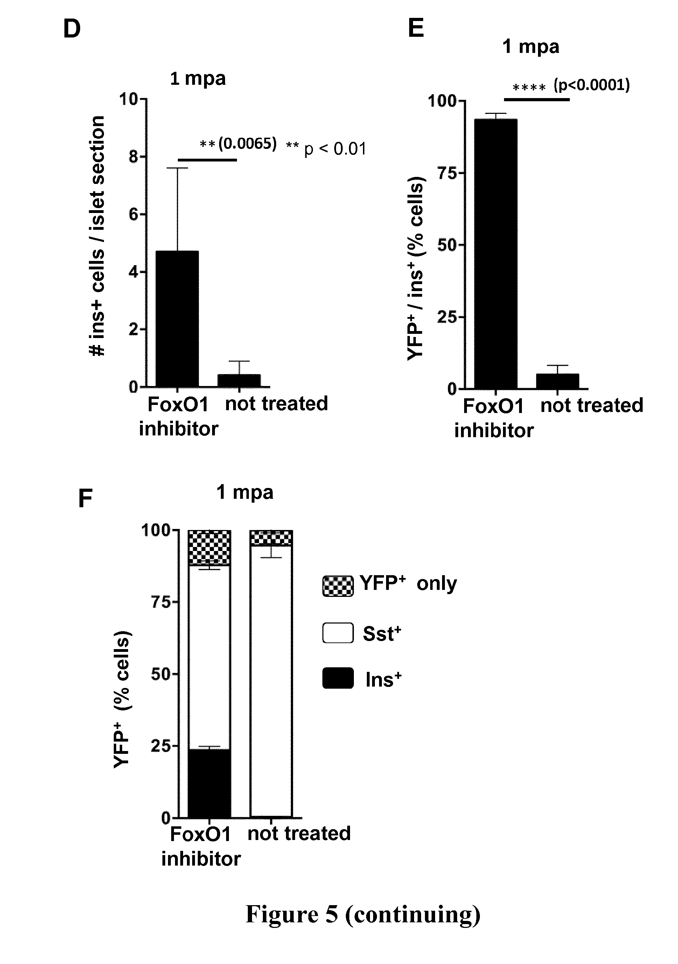

**Figure 5** reproduces conditions of induction of δ -cell conversion in diabetic adults. Tables depicting the transcriptional levels of **(A)** cell cycle regulators and PI3K/AKT/FoxO1 regulatory network genes, as well as **(B)** selected TGFβ pathway components and BMP pathway downstream effectors, in adults and pups δ cells at 1-week post-DT, as compared to their aged matched controls, **(C)** Design of transient FoxO1 activity inhibition in β-cell-ablated adult mice, **(D)** insulin⁺ cells in FoxO1 inhibitor-treated mice, **(E)** insulin⁺ YFP⁺ cells in FoxO1 inhibitor-treated mice, **(F)** YFP⁺ cells in adult β-cell-ablated and FoxO1-inhibited mice and proportion of insulin expressing cells (Ins⁺), somatostatin expressing cells (Sst⁺).

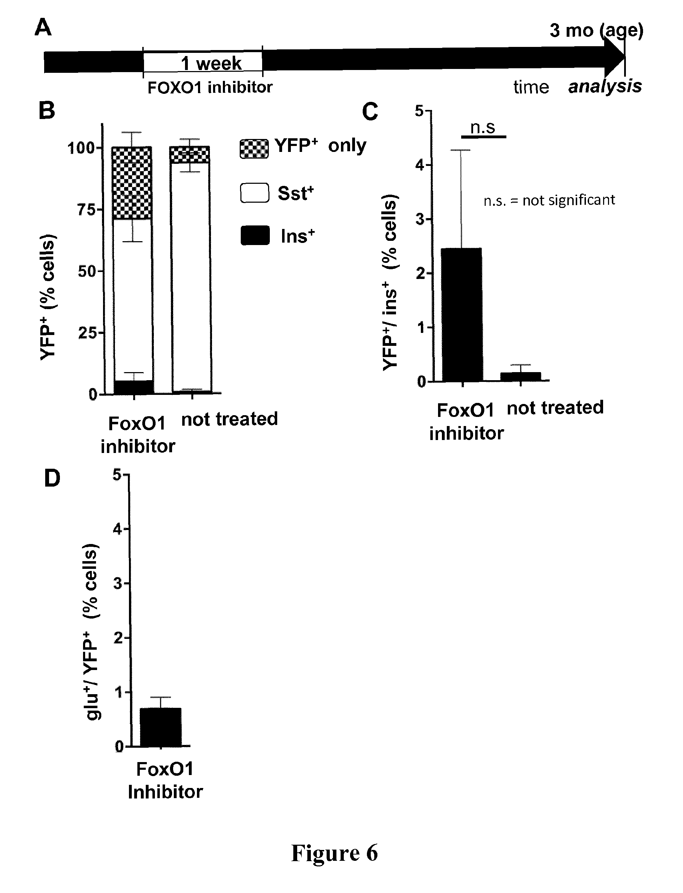

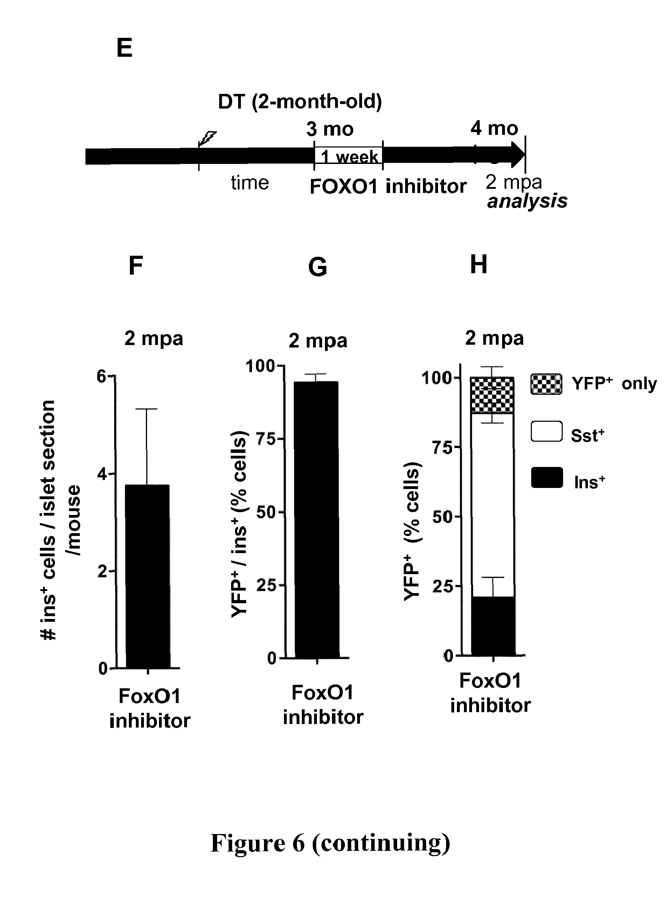

**Figure 6** shows that δ-cells de-differentiate in adult mice upon transient FoxO1 inhibition and, if in a situation of β-cell loss, they express insulin. FoxO1 inhibition by the compound AS1842856 for 1 week. **(A)** Scheme depicting the FoxO1 administration in non-ablated control SomatostatinCre, R26YFP, RIPDTR mice, **(B)** fate of the YFP⁺ labelled cells with and without FoxO1 inhibitor, **(C)** percentage of insulin⁺ cells labelled with YFP in treated and non-treated mice, **(D)** percentage of glucagon⁺ cells labelled with YFP in treated mice, **(E)** scheme depicting the clinically relevant FoxO1 transient inhibition 1 month after β-cell ablation, **(F)** number of insulin cells per islet section in 2 mpa ablated adults following AS 1842856 administration, **(G)** percentage of insulin⁺ cells labelled with YFP, **(H)** fate of the YFP⁺ labelled cells following FoxO1 treatment at 1 mpa.

### DETAILED DESCRIPTION OF THE INVENTION

As used herewith, "δ-cells", "delta cells" and "D cells" are somatostatin-producing cells, which can be found in the stomach, intestine and the islets of Langerhans in the pancreas. δ-cells make up approximately 10% of the cells in human pancreatic islets *(*Brissova et al, 2005, J. Histochem. Cytochem. 53(9):1087-1097*).*

As used herewith, "β-cells" or "beta cells" are a type of cells in the pancreas located in the islets of Langerhans of the pancreas, which make up approximately 54% of the cells in human islets *(Brissova et al, 2005, supra).* The primary function of β-cells is to manufacture, store and release insulin, a hormone that brings about effects which reduce blood glucose concentration. β-cells can respond quickly to transient increases in blood glucose concentrations by secreting some of their stored insulin while simultaneously producing more.

As used herewith, "α-cells" or "alpha cells" are endocrine cells in the islets of Langerhans of the pancreas, which make up approximately 35% of the human islet cells *(Brissova et al, 2005, supra)* and are responsible for synthesizing and secreting the peptide hormone glucagon, which elevates the glucose levels in the blood.

The "Forkhead box protein O1", also called "Forkhead in rhabdomyosarcoma" (generally abbreviated as "FoxO1" in mice or "FOXO1" in humans) is a protein that in humans is encoded by the *FOXO1* gene. In mice, the FoxO1 protein has 652 amino acids, its sequence is that disclosed under Genebank accession number (EDL35224.1) (SEQ ID NO: 1) and is encoded by a gene of sequence disclosed under Genebank accession number NM_019739.3 (SEQ ID NO: 3). In humans, FOXO1 protein has 655 amino acids, its amino acid sequence is that disclosed under Genebank accession number AAH70065.3 (SEQ ID NO: 2) and is encoded by a gene of sequence disclosed under Genebank accession number NM_002015.3 (SEQ ID NO: 4). As used herewith, the term "FOXO1" designates the Forkhead box protein O1 from any species, in particular human or murine. As used herein, the term FOXO1 also encompasses species variants, homologues, substantially homologous variants (either naturally occurring or synthetic), allelic forms, mutant forms, and equivalents thereof, including conservative substitutions, additions, deletions therein not adversely affecting the structure or function of the protein. FOXO1 is a transcription factor that plays important roles in regulation of gluconeogenesis and glycogenolysis by insulin signaling, and is also central to the decision for a preadipocyte to commit to adipogenesis. FOXO1 is primarily regulated through phosphorylation on multiple residues; its transcriptional activity is primarily dependent on its phosphorylation state. FOXO family proteins function primarily as transcription factors in the cell nucleus and bind to their cognate DNA targeting sequences. Therefore, they can regulate cell fate by modulating the expression of genes involved in apoptosis, oxidative detoxification, longevity, DNA repair, cell cycle transitions, glucose metabolism, energy homeostasis, cell differentiation, control of muscle growth *(*Greer and Brunet, 2005, Oncogene, 24(50):7410-25*;* Huang and Tindall, 2007, Journal of Cell Science 120:2479-2487*).* Moreover, FOXO family transcription factors can bind to specific binding partners allowing for a broad transcriptional response (Table 1 of van der Vos and Coffer, 2008, Oncogene 27, 2289-2299*).* In addition, FOXO family transcription factors are controlled by signalling networks that respond to external factors (*Huang and Tindall, 2007, supra).*

The term "homologous", applied to a gene variant or a polypeptide variant, means a gene variant or a polypeptide variant substantially homologous to a gene or a polypeptide of reference, but which has a nucleotide sequence or an amino acid sequence different from that of the gene or polypeptide of reference, respectively, being either from another species or corresponding to natural or synthetic variants as a result of one or more deletions, insertions or substitutions. Substantially homologous means a variant nucleotide sequence that is at least 80%, at least 85%, at least 90%, at least 95%, at least 96%, at least 97%, at least 98% or at least 99% identical to the nucleotide sequence of a gene of reference or an equivalent gene, i.e. exerting the same function, in another species. Substantially homologous means a variant amino acid sequence that is at least 80%, at least 85%, at least 90%, at least 95%, at least 96%, at least 97%, at least 98% or at least 99% identical to the amino acid sequence of a polypeptide of reference or an equivalent polypeptide, i.e. exerting the same function, in another species. The percentage of identity between two amino acid sequences or two nucleic acid sequences can be determined by visual inspection and/or mathematical calculation, or more easily by comparing sequence information using a computer program such as Clustal package version 1.83. Variants of a gene may comprise a sequence having at least one conservatively substituted amino acid, meaning that a given amino acid residue is replaced by a residue having similar physiochemical characteristics.

The term "FOXO1 inhibitors" or "FOXO1 antagonists" defines herewith a molecule that inhibits completely or partially the activity of a biological molecule, in the present context the activity of FOXO1 protein by directly targeting the FOXO1 protein and/or targeting its binding partners, its target genes or the signalling networks controlling FOXO expression. FOXO1 inhibitors or FOXO1 antagonists may include direct inhibitors of FOXO1 activity as well as modulators of FOXO family binding partners (including the androgen receptor, estrogen receptor and smad3), modulators of FOXO family target genes (including p15, p21 and p27) and modulators of the signalling networks controlling FOXO family expression (including Skp2). Thus, the term "FOXO1 inhibitor" is intended to include, but is not limited to, molecules which neutralize the effect of FOXO1, in particular its function as a transcription factor. FOXO binding partners include: androgen receptor, β-catenin, constitutive androstane receptor, Cs1, C/EBPα, C/EPBβ, estrogen receptor, FoxG1, FSH receptor, HNF4, HOXA5, HOXA10, myocardin, PGC-1α, PPARα, PPARγ, PregnaneX receptor, progesterone receptor, retinoic acid receptor, RUNX3, smad3, smad4, STAT3, thyroid hormone receptor (van der Vos and Coffer, 2008, Oncogene 27:2289-2299*).* FOXO family target genes include: BIM-1, bNIP3, Bcl-6, FasL, Trail (cell death), catalase, MnSOD, PA26 (detoxification); GADD45, DDB1 (DNA repair), p27KIP1, GADD45, p21CIP1, p130, Cyclin G2 (cell cycle arrest), G6Pase, PEPCK (glucose metabolism), NPY, AgRP (energy homeostasis), BTG-1, p21CIP1 (differentiation), atrogin-1 (atrophy) *(*Greer and Brunet, 2005, Oncogene, 24(50):7410-25*).* Modulators of signalling networks controlling FOXO expression include Skp2 (Huang and Tindall, 2007, Journal of Cell Science 120: 2479-248*).* For example, FOXO1 inhibitors may include small molecules, peptides, peptidomimetics, chimeric proteins, natural or unnatural proteins, nucleic acids or nucleic acid derived polymers such as DNA and RNA aptamers, siRNAs (small interfering RNAs), shRNAs (short hairpin RNAs), anti-sense nucleic acid, microRNA (miRNA), or complementary DNA (cDNA), PNAs (Peptide Nucleic Acids), or LNAs (Locked Nucleic Acids), fusion proteins with FOXO1 antagonizing activities, antibody antagonists such as neutralizing anti-FOXO1 antibodies, or gene therapy vectors driving the expression of such FOXO1 inhibitors. For example, FOXO1 inhibitors include the molecules described in Nagashima et al, 2010 (Molecular pharmacology 78: 961-970*)* and Tanaka et al, 2010 (European journal of pharmacology 645:185-191*)* such as 5-amino-7-(cyclohexylamino)-1-ethyl-6-fluoro-4-oxo-1,4-dihydroquinoline-3-carboxylic acid (AS1842856), 1-cyclopentyl-6-fluoro-4-oxo-7-(tetrahydro-2H-pyran-3-ylamino)-1,4-dihydro-quinoline-3-carboxylic acid (AS1841674), 7-(cyclohexylamino)-6-fluoro-4-oxo-1-(prop-1-en-2-yl)-1,4-dihydroquinoline-3-carboxylic acid (AS1838489), 7-(cyclohexylamino)-6-fluoro-1-(3-fluoroprop-1-en-2-yl)-4-oxo-1,4-dihydroquinoline-3-carboxylic acid (AS1837976), 7-(cyclohexylamino)-1-(cyclopent-3-en-1-yl)-6-fluoro-4-oxo-1,4-dihydro-quinoline-3-carboxylic acid (AS1805469) and 7-(cyclohexylamino)-6-fluoro-5-methyl-4-oxo-1-(pentan-3-yl)-1,4-dihydroquinoline-3-carboxylic acid (AS1846102), as well as small interfering RNA (siRNA), short hairpin RNA (shRNA). Examples of siRNAs or shRNAs targeting FOXO1 include siRNA #6242 (Alikhani et al., 2005, J. Biol. Chem. 280: 12096-12102*)* and examples of antibodies directed against FOXO1 include antibody #9454 (Kanao et al., 2012, PloS ONE 7(2), e30958*),* antibodies H128 and ac11350 *(*Liu et al., PLoS ONE 8(2), e58913*).* FOXO1 inhibitors also include molecules which inhibit the proper nuclear localization of FOXO1 such as, for instance, proteins encoded by any one of the genes selected from the group consisting of: serum/glucocorticoid regulated kinase (Accession No.: BC016616), FK506 binding protein 8 (Acc. No.: BC003739), apolipoprotein A-V (Acc. No.: BC011198), stratifin (Acc. No.: BC000995), translocation protein 1 (Acc. No.: BC012035), eukaryotic translation elongation factor 1 alpha 1 (Acc. No.: BC010735), lymphocyte cytosolic protein 2 (Acc. No.: BC016618), sulphide quinone reductase-like (Acc. No.: BC011153), serum/glucocorticoid regulated kinase-like (Acc. No.: BC015326), tyrosine 3-monooxygenase/tryptophan 5-monooxygenase activation protein, zeta polypeptide (Acc. No.: BC003623), tyrosine 3-monooxygenase/tryptophan 5-monooxygenase activation protein, gamma polypeptide (Acc. No.: BC020963) as described in Table 2 of US 2009/0156523.

In some embodiments of the invention, FOXO1 inhibitors can be in a form targeting pancreatic islets or δ-cells. As defined herewith, a FOXO1 inhibitor targets pancreatic islets if said inhibitor is preferentially transported to, or retained in, the pancreatic islets where it can exert its inhibitory effect with a higher efficiency than in other organs. As defined herewith, a FOXO1 inhibitor targets δ-cells if said inhibitor is preferentially attached to, or penetrates a δ-cell, where it can exert its inhibitory effect on FOXO1 expression and/or activity with a higher efficiency than it penetrates and/or exerts its inhibitory effect in a non-δ-cell such as a pancreatic α-cell or β-cell, a pancreatic polypeptide producing cell (PP cell), a ε-cell, or a neuroendocrine cell from the liver. Different standard methods in the art can be used to allow small molecule-, peptide-, protein- or nucleic acid- based FOXO1 inhibitors to target pancreatic islets or δ-cells. These methods include peptide mediated targeting of the islets of Langerhans *(*K.N Samli et al., 2005, Diabetes, 54:2103-2108*),* islet-targeting nanoparticles *(*Ghosh et al., 2012, Nano Lett. 12:203-208*),* liposomes or carbon nanotubes *(*Yu, 2010, Biochim Biophys Acta. 1805:97*).* Specific cells may be targeted using appropriate cell-specific ligands *(*Wang et al., 2012, NanoMedicine 9(2):3013-330*),* or specific subcellular organelles *(*Mossalem et al., 2010, Ther. Deliv. 1(1):169-193*).*

The terms "β-cell ablation" designate herewith the loss of β-cells, either total or partial, in the pancreas by apoptosis or necrosis as obtained using, for instance, diphtheria toxin and streptozotocin, respectively. Massive β-cell ablation can be obtained by homozygous transgenic expression of the diphtheria toxin receptor followed by administration of diphtheria toxin as disclosed in Naglich et al (cell, 1992, 69(6):1051-1061*)* or Saito et al (Nat Biotechnol, 2001, 19(8):746-750*).* Partial β-cell ablation can be obtained by heterozygous transgenic expression of the diphtheria toxin receptor flowed by administration of diphtheria toxin as above, or by using streptozotocin as disclosed in Lenzen (Diabetologia, 2008; 51: 216-26*).*

As used herewith the term "diabetes" refers to the chronic disease characterized by relative or absolute deficiency of insulin that results in glucose intolerance. This term covers diabetes mellitus, a group of metabolic diseases in which a person has high blood sugar level. As used herewith the term "diabetes" includes "diabetes mellitus type 1", a form of diabetes mellitus that results from autoimmune destruction of insulin-producing β cells of the pancreas, "diabetes mellitus type 2", a metabolic disorder that is characterized by high blood glucose in the context of insulin resistance and relative insulin deficiency, "gestational diabetes", a condition in which women without previously diagnosed diabetes exhibit high blood glucose levels during pregnancy, "neonatal diabetes", a rare form of diabetes that is diagnosed under the age of six months caused by a change in a gene which affects insulin production and "maturity onset diabetes of the young" (MODY), a rare form of hereditary diabetes caused by a mutation in a single gene. As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease.

The term "treatment" as used herein covers any treatment of a disease in a mammal, particularly a human, and includes: (a) preventing the disease from occurring in a subject who may be predisposed to the disease but has not yet been diagnosed as having it such as a preventive early asymptomatic intervention; (b) inhibiting the disease, i.e., arresting its development; or relieving the disease, i.e., causing regression of the disease and/or its symptoms or conditions such as improvement or remediation of damage. In particular, the methods, uses, formulations and compositions according to the invention are useful in the treatment of diabetes and/or in the prevention of evolution of diabetes. When applied to diabetes, prevention of a disease or disorder includes the prevention of the appearance or development of diabetes in an individual identified as at risk of developing diabetes, for instance due to past occurrence of diabetes in the circle of the individual's relatives or to the observation of risk factors including age, genetic factors, obesity, lifestyle, etc. Also covered by the terms "prevention/treatment" of diabetes is the stabilization of an already diagnosed diabetes in an individual. By "stabilization", it is meant the prevention or delay of evolution of diabetes leading to complications such as diabetic ketoacidosis, hyperosmolar non-ketotic state, hypoglycemia, diabetic coma, respiratory infections, periodontal disease, diabetic cardiomyopathy, diabetic nephropathy, diabetic neuropathy, diabetic foot, diabetic retinopathy, coronary artery disease, diabetic myonecrosis, peripheral vascular disease, stroke, diabetic encephalopathy.

The term "subject" as used herein refers to mammals. For examples, mammals contemplated by the present invention include human, primates, domesticated animals such as cattle, sheep, pigs, horses, laboratory rodents and the like. The term "subject" covers juvenile individuals as well as adults. In particular, the subjects can be juvenile or adult subjects suffering from, or at risk of developing, any form of diabetes where enhancement of insulin producing cell capability is a desirable therapeutic action.

The term "effective amount" as used herein refers to an amount of at least one FOXO1 inhibitor, composition or pharmaceutical formulation thereof according to the invention, as well as of isolated pancreatic islets or δ-cells according to the invention, that elicits the biological or medicinal response in a cell, tissue, system, animal or human that is being sought. In one embodiment, the effective amount is a "therapeutically effective amount" for the alleviation of the symptoms of the disease or condition being treated. In another embodiment, the effective amount is a "prophylactically effective amount" for prophylaxis of the symptoms of the disease or condition being prevented. The term also includes herein the amount of active FOXO1 inhibitor sufficient to delay the onset, or reduce the progression of the disease, notably to delay, reduce or inhibit the complications of diabetes thereby eliciting the response being sought (i.e. an "inhibition effective amount").

The term "efficacy" of a treatment according to the invention can be measured based on changes in the course of disease in response to a use or a method according to the invention. For example, the efficacy of a treatment of diabetes can be measured by a stable controlled glucose blood level, and/or periodic monitoring of glycated hemoglobin blood level.

The term "pharmaceutical formulation" refers to preparations which are in such a form as to permit biological activity of the active ingredient(s) to be unequivocally effective and which contain no additional component which would be toxic to subjects to which the said formulation would be administered.

### Methods of inducing insulin production in cells according to the invention

In a first aspect, the invention provides a method of inducing de-differentiation of δ-cells comprising the step of inhibiting FOXO1 expression and/or activity in said δ-cells.

In another aspect, the method of the invention relates to a method of converting de-differentiated δ-cells into insulin producing cells comprising the step of inhibiting FOXO1 expression and/or activity in said de-differentiated δ-cells.

The combination of the two steps of, first, de-differentiation of δ-cells and, second, conversion of de-differentiated δ-cells into insulin producing cells results in the conversion of δ-cells into insulin-producing cells.

Thus, in a general aspect, the invention provides a method of inducing insulin production in δ-cells comprising the step of inhibiting FOXO1 expression and/or activity in said δ-cells. The invention also provides a method of converting δ-cells into insulin producing cells, said method comprising the step of inhibiting FOXO1 expression and/or activity in said δ-cells. One skilled in the art will understand that once said δ-cells have been treated according to the invention to produce insulin, they are not stricto senso "δ-cells" any more (for instance they will have stopped producing somatostatin, which is characteristic of δ-cells) but this term is used herewith to indicate that said insulin-producing cells derive from δ-cells.

In a particular embodiment of the invention, inhibition of FOXO1 preferentially occurs in pancreatic islets or more preferentially in δ-cells and not, or only in a limited or undetectable amount, in other organs such as the liver, or in non-δ-cell types such as pancreatic α-cells, β-cells, pancreatic polypeptide producing cells (PP cells), ε-cells, or neuroendocrine cells from the liver, for instance.

In one embodiment, said δ-cells are from the pancreas, in particular from the islets of Langerhans from the pancreas, herewith also called pancreatic islets.

In another embodiment, said δ-cells are from gastrointestinal tissues including stomach and intestine.

In one embodiment, said pancreatic islets or δ-cells are from a juvenile or from an adult diabetic subject.

In another embodiment, said pancreatic islets or δ-cells are from a juvenile or adult subject predisposed to diabetes but who has not yet been diagnosed as having it for example based on familial history or on risk factors.

In a particular embodiment, said δ-cells are gastrointestinal δ-cells from an adult subject.

In another particular embodiment, said δ-cells are gastrointestinal δ-cells from a subject suffering from, or at risk of suffering from, diabetes.

In a still other embodiment, after extraction from the subject, in particular when the pancreatic islets or cells have been extracted from an adult, said pancreatic islets or δ-cells are submitted to a culture medium replicating similar conditions as those found in a juvenile physiological environment. Said culture medium may, for example, be characterized by the complete absence of adult sex steroids.

The methods of the invention can be applied *ex vivo* on isolated cells, cell cultures, tissues or sections thereof including those comprising islets of Langerhans from the pancreas or gastrointestinal tissue, or *in vivo* in the whole body of an animal, in particular a human subject, or a non-human mammal such as a laboratory rodent, for instance a mouse.

In an alternative particular embodiment of the invention, the inhibition of FOXO1 preferentially occurring in δ-cells is obtained by contacting, *ex vivo,* a FOXO1 inhibitor with a population of δ-cells, or with a population of pancreatic or gastrointestinal cells containing a significant number of δ-cells, e.g. where at least 5% or at least 10% of the cells are δ-cells. Different standard methods in the art allow isolation of δ-cells from pancreatic tissue and/or gastrointestinal tissues. Pancreatic tissue and islet cells comprising δ-cells can be isolated according to standard methods in the art including fluorescence activated cell sorting (FACS) of human islet cells as described in Dorrell et al. (2011, Diabetologia, 54:2832-2844*),* or Bramswig et al (2013, J. Clin. Inv. 123:1275-1284*).*

In one embodiment, the method of the invention relates to an *ex vivo* method of inducing insulin production in δ-cells comprising the steps of:
- obtaining a population of δ-cells from a subject, in particular a mammalian subject;
- optionally submitting said population of δ-cells to a medium replicating similar conditions as those found in a juvenile physiological environment;
- contacting, *ex vivo,* said population of δ-cells with at least one FOXO1 inhibitor, thereby generating insulin-producing cells;
- optionally, collecting said insulin-producing cells.

In one embodiment, the method of the invention relates to an *ex vivo* method of converting δ-cells into insulin producing cells, comprising the steps of:
- obtaining a population of δ-cells from a subject, in particular a mammalian subject;
- optionally submitting said population of δ-cells to a medium replicating similar conditions as those found in a juvenile physiological environment;
- contacting, *ex vivo,* said population of δ-cells with at least one FOXO1 inhibitor, thereby inducing de-differentiation and re-differentiation into insulin-producing cells;
- optionally, collecting said insulin-producing cells.

In one embodiment, the inhibition of FOXO1 in δ-cells, *ex vivo,* is obtained by contacting said δ-cells, present as isolated δ-cells or within isolated pancreatic islets, with at least one FOXO1 inhibitor, in particular any one of those described herewith, optionally in a form allowing targeting of δ-cells or pancreatic islets.

In another specific embodiment, the method of the invention relates to a method of inducing insulin production in δ-cells in a subject, in particular a mammalian subject, comprising administering to said subject at least one FOXO1 inhibitor, in particular any one of those described herewith, in a form allowing targeting pancreatic islets or δ-cells.

In another specific embodiment, the method of the invention relates to a method of converting δ-cells into insulin producing cells in a subject, in particular a mammalian subject, comprising administering to said subject at least one FOXO1 inhibitor, in particular any one of those described herewith, in a form allowing targeting pancreatic islets or δ-cells.

In one particular embodiment, the FOXO1 inhibitor is targeted to pancreatic islets using an islet-specific ligand.

In another particular embodiment, the FOXO1 inhibitor is targeted to δ-cells using a δ-cell specific ligand.

For instance, said ligand can be present at the surface of a nanoparticle, a liposome, or a nanotube, into which said FOXO1 inhibitor has been loaded, or be conjugated to the FOXO1 inhibitor itself

In one embodiment of the invention, said FOXO1 inhibitor is selected from the group consisting of: small molecules, peptides, peptidomimetics, chimeric proteins, natural or unnatural proteins, nucleic acids or nucleic acid derived polymers such as DNA and RNA aptamers, siRNAs (small interfering RNAs), shRNAs (short hairpin RNAs), anti-sense nucleic acid, microRNA (miRNA), complementary DNA (cDNA), PNAs (Peptide Nucleic Acids), or LNAs (Locked Nucleic Acids), fusion proteins with FOXO1 antagonizing activities, antibody antagonists such as neutralizing anti-FOXO1 antibodies, or gene therapy vectors driving the expression of such FOXO1 inhibitors.

In a further embodiment of the invention, said FOXO1 inhibitor is a small molecule selected from the group consisting of: 5-amino-7-(cyclohexylamino)-1-ethyl-6-fluoro-4-oxo-1,4-dihydroquinoline-3-carboxylic acid (AS1842856), 1-cyclopentyl-6-fluoro-4-oxo-7-(tetrahydro-2H-pyran-3-ylamino)-1,4-dihydro-quinoline-3-carboxylic acid (AS1841674), 7-(cyclohexylamino)-6-fluoro-4-oxo-1-(prop-1-en-2-yl)-1,4-dihydroquinoline-3-carboxylic acid (AS1838489), 7-(cyclohexylamino)-6-fluoro-1-(3-fluoroprop-1-en-2-yl)-4-oxo-1,4-dihydroquinoline-3-carboxylic acid (AS1837976), 7-(cyclohexylamino)-1-(cyclopent-3-en-1-yl)-6-fluoro-4-oxo-1,4-dihydro-quinoline-3-carboxylic acid (AS1805469) and 7-(cyclohexylamino)-6-fluoro-5-methyl-4-oxo-1-(pentan-3-yl)-1,4-dihydroquinoline-3-carboxylic acid (AS1846102).

In a particular embodiment, said FOXO1 inhibitor is 5-amino-7-(cyclohexylamino)-1-ethyl-6-fluoro-4-oxo-1,4-dihydroquinoline-3-carboxylic acid.

In a further embodiment of the invention, said FOXO1 inhibitor is a silencing nucleic acid such as a siRNA, shRNA or antisense oligonucleotide, specific for FOXO1.

In another embodiment of the invention, said FOXO1 inhibitor is a neutralizing antibody such as a polyclonal antibody or a monoclonal antibody with specificity to FOXO1.

As mentioned above, in another embodiment of the invention, said FOXO1 inhibitor can be loaded into a nanoparticle, a liposome, or a nanotube, which comprises an islet-specific ligand and/or a δ-cell specific ligand at its surface.

In an alternative embodiment, said FOXO1 inhibitor can be coupled (e.g. by covalent binding or non-covalent binding) to an islet-specific ligand and/or a δ-cell specific ligand.

In a specific embodiment of the invention, said FOXO1 inhibitor targeting pancreatic islets or δ-cells comprises a nanoparticle comprising a surface ligand directed to a pancreatic islet or δ-cell specific marker, loaded with a FOXO1 inhibitor as defined herewith. Such surface ligand can for example, be a binding partner to a cell surface receptor, or an antibody directed to a specific cell surface epitope.

In a particular embodiment of the invention, when said FOXO1 inhibitor is a peptide, polypeptide, protein, or a nucleic acid such as a siRNA or a shRNA, said FOXO1 inhibitor is administered to said subject or placed in contact with said δ-cells by transfecting δ-cells with a nucleic acid comprising the coding sequence of said FOXO1 inhibitor's gene or a nucleic acid encoding said siRNA or shRNA, placed under the control of a constitutive or inducible promoter.

In the method of the invention, the nucleic acid for transfecting said δ-cells is in the form of a vector (either a viral or non-viral vector) and is delivered into said cells using standard methods in the art including microbubbles, calcium phosphate-DNA co-precipitation, DEAE-dextran-mediated transfection, polybrene-mediated transfection, electroporation, microinjection, liposome fusion, lipofection, protoplast fusion, retroviral infection, and biolistics.

In a further embodiment of the methods of the invention, at least 10%, in particular at least 20%, more particularly at least 30%, even more particularly at least 40% of the cells obtained with said methods are insulin producing cells.

In another embodiment of the methods of the invention, the amount of insulin produced by the cells obtained with said methods is sufficient to render a significant improvement in the subject's ability to control blood glucose levels. Blood glucose measurement methods are well-known to those skilled in the art.

In a specific embodiment, the methods of the invention further comprise the step of β-cells ablation (partial or total) in the pancreatic islets of the tissue comprising said δ-cells, either at the tissue level or *in vivo,* using, for instance, transgenic expression of the diphtheria toxin receptor followed by administration of diphtheria toxin as disclosed in Naglich et al (cell 1992, 69(6): 1051-1061*)* or Saito et al (Nat Biotechnol, 2001, 19(8): 746-750*),* or by using streptozotocin as disclosed in Lenzen (Diabetologia, 2008; 51: 216-226*).*

### Methods of treatment and uses according to the invention

Another aspect of the invention relates to a method of preventing and/or treating diabetes comprising the administration of a therapeutically effective amount of at least one FOXO1 inhibitor targeting pancreatic islets or δ-cells in a subject in need thereof.

In one embodiment, said FOXO1 inhibitor in a form allowing targeting of pancreatic islets or δ-cells comprises a nanoparticle comprising a surface ligand directed to a pancreatic islet or δ-cell specific marker, loaded with a FOXO1 inhibitor as defined herewith.

In a specific embodiment of the method of the invention, said FOXO1 inhibitor is selected from the group consisting of: 5-amino-7-(cyclohexylamino)-1-ethyl-6-fluoro-4-oxo-1,4-dihydroquinoline-3-carboxylic acid (AS1842856), 1-cyclopentyl-6-fluoro-4-oxo-7-(tetrahydro-2H-pyran-3-ylamino)-1,4-dihydro-quinoline-3-carboxylic acid (AS1841674), 7-(cyclohexylamino)-6-fluoro-4-oxo-1-(prop-1-en-2-yl)-1,4-dihydroquinoline-3-carboxylic acid (AS1838489), 7-(cyclohexylamino)-6-fluoro-1-(3-fluoroprop-1-en-2-yl)-4-oxo-1,4-dihydroquinoline-3-carboxylic acid (AS1837976), 7-(cyclohexylamino)-1-(cyclopent-3-en-1-yl)-6-fluoro-4-oxo-1,4-dihydro-quinoline-3-carboxylic acid (AS1805469) and 7-(cyclohexylamino)-6-fluoro-5-methyl-4-oxo-1-(pentan-3-yl)-1,4-dihydroquinoline-3-carboxylic acid (AS1846102), in a form allowing targeting of pancreatic islets or δ-cells.

In an alternative embodiment of the method of the invention, said FOXO1 inhibitor is selected from the group consisting of: nucleic acid derived polymers such as DNA and RNA aptamers, siRNAs (small interfering RNAs), shRNAs (short hairpin RNAs), PNAs (Peptide Nucleic Acids), or LNAs (Locked Nucleic Acids), fusion proteins with FOXO1 antagonizing activities, antibody antagonists such as neutralizing anti-FOXO1 antibodies, or gene therapy vectors driving the expression of such FOXO1 inhibitors, in a form allowing targeting of pancreatic islets or δ-cells.

In a particular embodiment of the invention, said FOXO1 inhibitor is a silencing nucleic acid such as a siRNA, shRNA or antisense oligonucleotide, specific for FOXO1.

In another particular embodiment of the invention, said FOXO1 inhibitor is a neutralizing antibody such as a polyclonal antibody or a monoclonal antibody with specificity to FOXO1. In a specific embodiment, the method of preventing and/or treating diabetes according to the invention comprises auto-grafting or allo-grafting of δ-cells (e.g. pancreatic and/or gastrointestinal δ-cells), converted into insulin-producing cells by contacting said δ-cells with at least one FOXO1 inhibitor as described herewith.

Auto-grafting consists of grafting converted cells derived from δ-cells isolated from the subject to be treated, whereas allo-grafting consists of grafting converted cells derived from δ-cells isolated from a subject different from the subject to be treated but belonging to the same species.

δ-cells useful in the method of preventing and/or treating diabetes comprising auto-grafting or allo-grafting of δ-cells converted according to the method of the invention can be pancreatic δ-cells and/or δ-cells from the stomach or intestine.

Thus, another aspect of the invention relates to a method of preventing and/or treating diabetes comprising:
a) converting, *ex vivo,* δ-cells into insulin producing cells, comprising:
   - obtaining a population of δ-cells from a subject;
   - contacting said population of δ-cells with at least one FOXO1 inhibitor, thereby generating insulin-producing cells;
b) collecting said insulin-producing cells produced in step a);
c) introducing the insulin-producing cells collected in step b) into a subject in need thereof in a therapeutically effective amount.

In a particular embodiment, said method of preventing and/or treating diabetes further comprises submitting said population of δ-cells under a) to a medium replicating similar conditions as those found in a juvenile physiological environment before the step of contacting with at least one FOXO1 inhibitor and/or at the same time as contacting with at least one FOXO1 inhibitor.

In another particular embodiment, said method of preventing and/or treating diabetes further comprises determining the level of insulin produced by the cells collected in step b) and/or determining the number or percentage of cells collected in step b) which produce insulin.

In a further embodiment, said method of preventing and/or treating diabetes further comprises introducing the cells collected in step b) which produce insulin into a subject in need thereof.

Standard methods in the art allow determining the level of insulin. Such methods include ELISA (Kekow et al, 1988, Diabetes 37:321-326*;* Johansen et al, 1999, Journal of Endocrinology 162:87-93*)* and radioimmunoassay (Muscelli et al, 2001, International Journal of Obesity 25:798-804).

In one particular aspect of said method, said δ-cells are from a mammalian subject suffering from diabetes and, once converted into insulin-producing cells according to the method of the invention, are re-introduced in the same subject as the one from whom said δ-cells were obtained (e.g. as in autografting).

In another particular aspect of said method, said δ-cells are from a mammalian subject suffering from diabetes or not and, once converted into insulin-producing cells, are re-introduced in a different subject from the same species as the one from whom said δ-cells were obtained (e.g. as in allografting).

In another aspect, the invention provides a use of at least one FOXO1 inhibitor targeting pancreatic islets or δ-cells as described herewith in the manufacture of a medicament for preventing and/or treating diabetes.

In one embodiment of the use of the invention, FOXO1 inhibitor targeting pancreatic islets or δ-cells comprises nanoparticle comprising a surface ligand directed to a pancreatic islet or δ-cell specific marker, loaded with a FOXO1 inhibitor as defined herewith.

In a specific embodiment of the use of the invention, said FOXO1 inhibitor is selected from the group consisting of: 5-amino-7-(cyclohexylamino)-1-ethyl-6-fluoro-4-oxo-1,4-dihydroquinoline-3-carboxylic acid (AS 1842856), 1-cyclopentyl-6-fluoro-4-oxo-7-(tetrahydro-2H-pyran-3-ylamino)-1,4-dihydro-quinoline-3-carboxylic acid (AS1841674), 7-(cyclohexylamino)-6-fluoro-4-oxo-1-(prop-1-en-2-yl)-1,4-dihydroquinoline-3-carboxylic acid (AS1838489), 7-(cyclohexylamino)-6-fluoro-1-(3-fluoroprop-1-en-2-yl)-4-oxo-1,4-dihydroquinoline-3-carboxylic acid (AS1837976), 7-(cyclohexylamino)-1-(cyclopent-3-en-1-yl)-6-fluoro-4-oxo-1,4-dihydro-quinoline-3-carboxylic acid (AS1805469) and 7-(cyclohexylamino)-6-fluoro-5-methyl-4-oxo-1-(pentan-3-yl)-1,4-dihydroquinoline-3-carboxylic acid (AS1846102), more particularly 5-amino-7-(cyclohexylamino)-1-ethyl-6-fluoro-4-oxo-1,4-dihydroquinoline-3-carboxylic acid (AS1842856), and is loaded into a nanoparticle comprising a surface ligand directed to a pancreatic islet or δ-cell specific marker.

In another aspect, the invention provides a use of isolated pancreatic islets or isolated δ-cells comprising δ-cells converted into insulin producing cells according to the method of the invention in the manufacture of a medicament for preventing and/or treating diabetes.

In another embodiment, the use of at least one FOXO1 inhibitor targeting pancreatic islets or δ-cells is combined with the use of δ-cells, in particular pancreatic and/or gastrointestinal δ-cells, converted into insulin producing cells, for preventing and/or treating diabetes.

In another aspect of the invention are provided isolated pancreatic islets or isolated δ-cells comprising δ-cells converted into insulin producing cells as described herewith for use in preventing and/or treating diabetes.

In a still other aspect of the invention are provided FOXO1 inhibitors targeting pancreatic islets or δ-cells as described herewith for use in preventing and/or treating diabetes.

### Methods of screening according to the invention

In a still other aspect of the invention is provided a method of screening a compound for its ability to inhibit FOXO1 expression and/or activity comprising:
a) exposing isolated pancreatic islets or isolated δ-cells, comprising δ-cells expressing FOXO1, to a test compound;
b) determining the number of cells which are insulin producing cells in presence and in absence of the test compound;
c) comparing the two values of number of insulin producing cells determined in step b), wherein a number of insulin producing cells that is higher in presence of the test compound compared to the number determined in absence of the test compound is indicative of a test compound able to inhibit FOXO1 expression and/or activity.

Any known method may be used for the determination of the number of insulin producing cells, including immunofluorescent staining.

### Agents and compositions according to the invention

In one aspect, the invention provides isolated pancreatic islets or isolated δ-cells, comprising δ-cells converted into insulin producing cells by contacting said islets or δ-cells with at least one FOXO1 inhibitor, as well as a composition comprising said δ-cells converted into insulin producing cells.

In another aspect, the invention provides FOXO1 inhibitors targeting pancreatic islets or δ-cells as described herewith, as well as a composition comprising said FOXO1 inhibitors targeting pancreatic islets or δ-cells.

In another aspect of the invention are provided isolated pancreatic islets or isolated δ-cells comprising δ-cells converted into insulin producing cells as described herewith for use as a medicament.

In the sense of the invention, isolated δ-cells, optionally within isolated pancreatic islets, are also referred to as isolated pancreatic islets or isolated δ-cells, comprising δ-cells.

In a still other aspect of the invention are provided FOXO1 inhibitors targeting pancreatic islets or δ-cells as described herewith for use as a medicament.

In one embodiment of the above mentioned aspects, said FOXO1 inhibitor is selected from the group consisting of: small molecules, peptides, peptidomimetics, chimeric proteins, natural or unnatural proteins, nucleic acid derived polymers (such as DNA and RNA aptamers, siRNAs (small interfering RNAs), shRNAs (short hairpin RNAs), PNAs (Peptide Nucleic Acids), or LNAs (Locked Nucleic Acids), fusion proteins with FOXO1 antagonizing activities, antibody antagonists such as neutralizing anti-FOXO1 antibodies, or gene therapy vectors driving the expression of such FOXO1 inhibitors.

In a specific embodiment, said FOXO1 inhibitor is selected from the group consisting of 5-amino-7-(cyclohexylamino)-1-ethyl-6-fluoro-4-oxo-1,4-dihydroquinoline-3-carboxylic acid (AS1842856), 1-cyclopentyl-6-fluoro-4-oxo-7-(tetrahydro-2H-pyran-3-ylamino)-1,4-dihydro-quinoline-3-carboxylic acid (AS1841674), 7-(cyclohexylamino)-6-fluoro-4-oxo-1-(prop-1-en-2-yl)-1,4-dihydroquinoline-3-carboxylic acid (AS1838489), 7-(cyclohexylamino)-6-fluoro-1-(3-fluoroprop-1-en-2-yl)-4-oxo-1,4-dihydroquinoline-3-carboxylic acid (AS1837976), 7-(cyclohexylamino)-1-(cyclopent-3-en-1-yl)-6-fluoro-4-oxo-1,4-dihydroquinoline-3-carboxylic acid (AS1805469) and 7-(cyclohexylamino)-6-fluoro-5-methyl-4-oxo-1-(pentan-3-yl)-1,4-dihydroquinoline-3-carboxylic acid (AS1846102), more particularly 5-amino-7-(cyclohexylamino)-1-ethyl-6-fluoro-4-oxo-1,4-dihydroquinoline-3-carboxylic acid (AS1842856).

The developed formula of some FOXO1 inhibitors useful in the invention are as follows (source: Nagashima et al, 2010, Molecular pharmacology 78:961-970*)*:

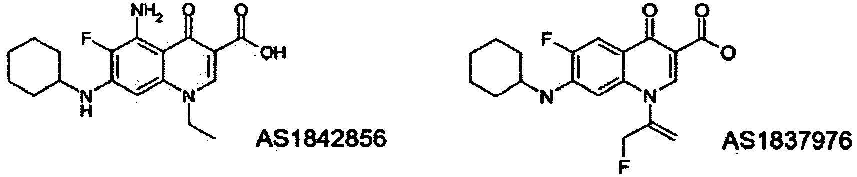

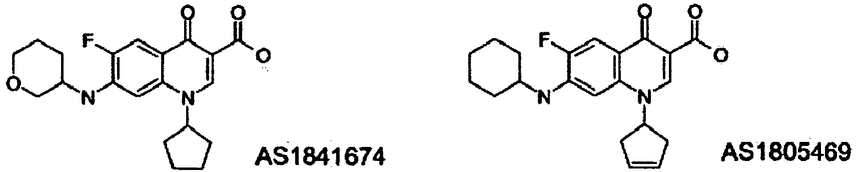

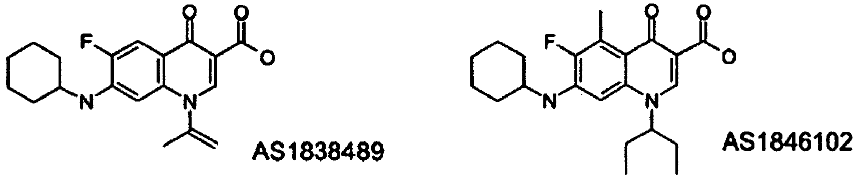

Methods to produce such FOXO1 inhibitors are known in the art.

In another specific embodiment, said FOXO1 inhibitor is a neutralizing antibody specific for FOXO1.

In a still further embodiment, said FOXO1 inhibitor is a silencing nucleic acid such as a siRNA, shRNA or antisense oligonucleotide, specific for FOXO1.

In a particular embodiment, the FOXO1 inhibitors are rendered capable of targeting pancreatic islets or δ-cells by combining said FOXO1 inhibitors with a ligand directed to a pancreatic islet or a δ-cell specific marker.

Examples of pancreatic islet specific markers include islet binding peptides (Samli et al, 2005, Diabetes 54(7):2103-2108*),* or islet vascular targeting (Yao et al, 2005, Am J Pathol. 166(2):625-36).

Examples of δ-cell specific markers may include antibodies raised to specific surface epitopes.

Thus, the invention also provides a FOXO1 inhibitor targeting pancreatic islets or δ-cells comprising a FOXO1 inhibitor and a ligand directed to a pancreatic islet or a δ-cell specific marker.

In a particular embodiment, FOXO1 inhibitors are rendered capable of targeting δ-cells whereas incapable of targeting non-δ-cells, or only in a limited extent, by loading a nanoparticle, liposome or nanotube, comprising a surface ligand directed to a δ-cell specific marker, with a FOXO1 inhibitor as described herewith. In an alternative embodiment, said FOXO1 inhibitors are coupled (e.g. through covalent or non-covalent binding) to a δ-cell specific marker.

In another embodiment of the invention, FOXO1 inhibitors are rendered capable of targeting pancreatic islets by loading a nanoparticle, liposome or nanotube, comprising a surface ligand directed to a pancreatic islet marker, with a FOXO1 inhibitor as described herewith. In an alternative embodiment, said FOXO1 inhibitors are coupled (e.g. through covalent or non-covalent binding) to a pancreatic islet marker.

The δ-cells useful in the invention include pancreatic δ-cells and gastrointestinal δ-cells.

In the invention, δ-cells can be from a diabetic subject or from a subject at risk of developing diabetes, or from a subject not at risk of developing diabetes.

In the invention, δ-cells can be from a juvenile or from an adult.

In a further embodiment, isolated pancreatic islets or isolated δ-cells comprising δ-cells converted into insulin producing cells are obtainable by the *ex vivo* method according to the invention.

In another embodiment, it is provided a composition comprising isolated pancreatic islets or isolated δ-cells comprising δ-cells converted into insulin producing cells as described herewith wherein at least 10%, in particular at least 20%, more particularly at least 30%, even more particularly at least 40% of said cells are insulin producing cells.

In a further embodiment, the invention provides pharmaceutical compositions and methods for treating a subject, preferably a mammalian subject, and most preferably a human subject who is at risk of, or suffering from, diabetes, said pharmaceutical composition comprising the FOXO1 inhibitors targeting pancreatic islets or δ-cells as described herewith and/or isolated pancreatic islets or isolated δ-cells comprising δ-cells converted into insulin producing cells as described herewith.

In one aspect, the invention provides FOXO1 inhibitors targeting pancreatic islets or δ-cells for use in preventing and/or treating diabetes, as well as a composition comprising said FOXO1 inhibitors for use in preventing and/or treating diabetes.

In another aspect, the invention provides isolated pancreatic islets or isolated δ-cells comprising δ-cells converted into insulin producing cells as described herewith for use in preventing and/or treating diabetes, as well as a composition comprising said δ-cells converted into insulin producing cells as described herewith for use in preventing and/or treating diabetes.

In another aspect, the invention provides isolated pancreatic islets or isolated δ-cells comprising δ-cells converted into insulin producing cells as described herewith for use in autografting or allografting for preventing and/or treating diabetes.

Pharmaceutical compositions or formulations according to the invention may be administered as a pharmaceutical formulation, which can contain an agent according to the invention in any form and/or δ-cells as described herewith.

The compositions according to the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous and intradermal) use by injection or continuous infusion. Injectable compositions are typically based upon injectable sterile saline or phosphate-buffered saline or other injectable carriers known in the art. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

Examples of suitable adjuvants include MPL® (Corixa), aluminum-based minerals including aluminum compounds (generically called Alum), ASO1-4, MF59, CalciumPhosphate, Liposomes, Iscom, polyinosinic:polycytidylic acid (polyIC), including its stabilized form poly-ICLC (Hiltonol), CpG oligodeoxynucleotides, Granulocyte-macrophage colony-stimulating factor (GM-CSF), lipopolysaccharide (LPS), Montanide, PLG, Flagellin, QS21, RC529, IC31, Imiquimod, Resiquimod, ISS, and Fibroblast-stimulating lipopeptide (FSL1). Compositions of the invention may be liquid formulations including, but not limited to, aqueous or oily suspensions, solutions, emulsions, syrups, and elixirs. The compositions may also be formulated as a dry product for reconstitution with water or other suitable vehicle before use. Such liquid preparations may contain additives including, but not limited to, suspending agents, emulsifying agents, non-aqueous vehicles and preservatives. Suspending agents include, but are not limited to, sorbitol syrup, methyl cellulose, glucose/sugar syrup, gelatin, hydroxyethyl cellulose, carboxymethyl cellulose, aluminum stearate gel, and hydrogenated edible fats. Emulsifying agents include, but are not limited to, lecithin, sorbitan monooleate, and acacia. Preservatives include, but are not limited to, methyl or propyl p-hydroxybenzoate and sorbic acid. Dispersing or wetting agents include but are not limited to poly(ethylene glycol), glycerol, bovine serum albumin, Tween®, Span®.

Compositions of the invention may also be formulated as a depot preparation, which may be administered by implantation or by intramuscular injection.

Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, acacia, gelatin, sorbitol, tragacanth, mucilage of starch and polyvinylpyrrolidone. Fillers include, but are not limited to, lactose, sugar, microcrystalline cellulose, maize starch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate.

Tablets may be coated according to methods well known in the art.

The compounds of this invention can also be administered in sustained release forms or from sustained release drug delivery systems.

According to a particular embodiment, compositions according to the invention are injectable for subcutaneous, intramuscular or intraperitoneal use or ingestable for oral use.

In another particular aspect, the compositions according to the invention are adapted for delivery by repeated administration.

Further materials as well as formulation processing techniques and the like are set out in Part 5 of Remington's "The Science and Practice of Pharmacy", 22nd Edition, 2012, University of the Sciences in Philadelphia, Lippincott Williams & Wilkins*,* which is incorporated herein by reference.

The dosage administered, as single or multiple doses, to an individual will vary depending upon a variety of factors, including pharmacokinetic properties, subject conditions and characteristics (sex, age, weight, body mass index (BMI), general health), extent of symptoms, concurrent treatments, frequency of treatment and the effect desired.

### Kits according to the invention

Another aspect of the invention provides a kit comprising material for carrying out any *ex vivo* method according to the invention.

In one embodiment, a kit for inducing insulin production in δ-cells and/or for converting δ-cells into insulin producing cells comprises:
(i) a cell culture medium replicating similar conditions as those found in a juvenile physiological environment;
(ii) at least one FOXO1 inhibitor, optionally in the form allowing targeting of pancreatic islets or δ-cells, as described herewith; and
(iii) optionally, isolated pancreatic islets or δ-cells.

According to a further embodiment, the kit according to the invention further comprises at least one reagent for quantification of insulin.

### Mode of administration

Compositions of this invention may be administered in any manner including intravenous injection, intra-arterial, intraperitoneal injection, subcutaneous injection, intramuscular, intra-thecal, oral route, cutaneous application, direct tissue perfusion during surgery or combinations thereof.

The compositions of this invention may also be administered in the form of an implant, which allows slow release of the compositions as well as a slow controlled i.v. infusion.

Delivery methods for the composition of this invention include known delivery methods for anti-diabetes drugs such as oral, intramuscular and subcutaneous.

### Combination

According to the invention, the agents and compositions according to the invention, and pharmaceutical formulations thereof can be administered alone or in combination with a co-agent useful in the treatment of diabetes such as insulin, biguanide, sulphonylureas, alpha glucosidase inhibitor, prandial glucose regulators, thiazolidinediones (glitazones), incretin mimetics, DPP-4 inhibitors (gliptins).

The invention encompasses the administration of an agent or composition according to the invention and pharmaceutical formulations thereof, wherein said agent or composition is administered to an individual prior to, simultaneously or sequentially with other therapeutic regimens, co-agents useful in the treatment of diabetes, in a therapeutically effective amount. An agent or composition according to the invention, or the pharmaceutical formulation thereof, that is administered simultaneously with said co-agents can be administered in the same or different composition(s) and by the same or different route(s) of administration. According to one embodiment, is provided a pharmaceutical formulation comprising an agent or composition according to the invention, combined with at least one co-agent useful in the treatment of diabetes, and at least one pharmaceutically acceptable carrier.

### Subjects

In an embodiment, subjects according to the invention are subjects suffering from diabetes. In a particular embodiment, subjects according to the invention are subjects suffering from diabetes mellitus type 1, diabetes mellitus type 2, gestational diabetes, neonatal diabetes, or maturity onset diabetes of the young (MODY).

In a further embodiment, subjects according to the invention are considered at risk for the development of diabetes mellitus type 1, diabetes mellitus type 2, gestational diabetes, neonatal diabetes, or maturity onset diabetes of the young (MODY). Such risk factors may include age, genetic factors, obesity, lifestyle, family antecedents, etc.

In a particular embodiment, subjects according to the invention are adults including young subjects having reached puberty.

In another embodiment, subjects according to the invention are juvenile subjects, i.e. subjects not yet capable of sexual reproduction.

In another particular embodiment, subjects according to the invention are subjects whose pancreatic β-cells decreased by more than 60% compared to non-diabetic subjects.

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the appended claims. The invention having been described, the following examples are presented by way of illustration, and not limitation.

### EXAMPLES

### Materials and methods

### Mice

RIP-DTR and Glucagon-rtTA *(*Thorel et al, 2010, Nature 464, 1149-1154*),* TetO-Cre *(*Perl et al, 2002, PNAS 99, 10482-10487), R26-EYFP (Srinivas et al, 2001, BMC developmental biology 1, 4*)*, R26-dTomato (Madisen et at, 2010, Nature neuroscience 13, 133-140*),* Ngn3-YFP (Mellitzer et al, 2004, Molecular endocrinology 18, 2765-2776), Ngn3-tTA and Tre-Ngn3 (Wang et al, 2009, PNAS 106, 9715-9720), R26-iDTR (Buch et al, 2005, Nature methods 2, 419-426), and Ngn3-CreERT (Gu et al, 2002, Development 129, 2447-2457*)* mice were previously described. The Somatostatin-Cre mice bears a Sst-mCherry-2A-iCre transgene. The Sst promoter was cloned from BAC bQ73b10, with NOD background; initially a rpsLneo sequence (Genebridges) providing kanamycin resistance and streptomycin sensitivity was introduced after the STOP codon in Sst-exon2 and subsequently all sequence between the Sst-START codon in exon1 and the rpsLneo sequence was replaced by the mCherry-2A-iCre sequence. In the resulting mice, no mCherry-fluorescence can be detected in tissue sections; however, when combined with the R26-EYFP or R26-dTomato transgenes, strong fluorescence can be detected in about 80% of pancreatic δ-cells as well as gastric D cells. In the Insulin-mCherry mice, more than 95% of insulin-expressing cells are mCherry⁺.

### Diphtheria toxin, tamoxifen, doxycycline, streptozotocin, FoxO1 inhibitor (AS1842856) and insulin treatments.

Diphtheria toxin (DT) (Sigma) was given in 3 intraperitoneal (i.p.) injections (126 ng of DT per injection, on days 0, 3 and 4), or as single intraperitoneal (i.p.) injection to 2-week-old pups. Injected middle-aged and aged mice were always males; pups of both genders were given DT, however only the males were used in the experiments presented here, for homogeneity.

Tamoxifen (TAM) was freshly prepared (Sigma) and administered i.p. (2 doses of 5 mg, 2 days apart). TAM (20 mg) was diluted in 50 µl 100% ethanol and 950 µl corn oil.

DOX (1 mg.ml-1) (Sigma) was added to drinking water for 2 weeks.

Streptozotocin (Sigma) was administrated by a single intra-peritoneal (i.p.) injection (200 mg/kg) to 2-week-old pups as previously described *(*Hu et al, 2011, Diabetes 60, 1705-1715*).* Either 30 mg/kg of FoxO1 inhibitor AS1842856 (Calbiochem) or the vehicle (DMSO) were i.p. injected daily, for 5 days, to 2-month-old mice.

Mice received subcutaneous implants of insulin (Linbit) when hyperglycemic (>20 mM) in the long-term regeneration experiments.

### Immunofluorescence

Cryostat tissue sections were prepared at 10 µm-thickness.

The following primary antibodies were used: guinea-pig anti-porcine insulin (Dako, 1/400), mouse anti-porcine glucagon (Sigma, 1/1,000), rabbit anti-human somatostatin (Dako, 1/200), mouse anti-human somatostatin (BCBC (Ab1985), 1/200), goat anti-human somatostatin (SantaCruz, 1/200), rabbit anti-human PP (Bachem, 1/200), mouse anti-Ki67 (BD Transduction Laboratory, 1/200), rabbit anti-GFP (Molecular Probes, 1/200), chicken anti-GFP (Abcam, 1/400), mouse anti-mCherry (Abcam, 1/500).

The secondary antibodies were as follows:
goat anti-mouse TRITC (IgG1 -γ1) (Southern Biotech, 1/500), goat anti-mouse 555 (IgG1 - γ1) (Molecular Probes, 1/500), goat anti-mouse 647 (IgG1 -γ1) (Molecular Probes, 1/500), goat anti-rabbit 488 (highly cross-adsorbed) (Molecular Probes, 1/500), donkey anti-rabbit 594 (Molecular Probes, 1/500), goat anti-chicken 488 (Molecular Probes, 1/500), goat anti-guinea pig 488 (highly cross-adsorbed) (Molecular Probes, 1/500), goat anti-guinea pig 488 (highly cross-adsorbed) (Molecular Probes, 1/500), goat anti-guinea pig 568 (highly cross-adsorbed) (Molecular Probes, 1/500), goat anti-guinea pig 647 (highly cross-adsorbed)

### (Molecular Probes, 1/500), donkey anti-goat 647 (Molecular Probes, 1/500).

The secondary antibodies were coupled with Alexa 488, 555, 546, 598 or 647 (Molecular Probes, 1:500), or TRITC (Southern Biotech, 1:500). Wherever necessary the secondary detection was performed in two sequential stages (we detected firstly the primary antibody raised in goat by using a donkey anti-goat AlexaFluor secondary antibody then, following extensive washings, we performed a second round of detection using a cocktail of the goat-raised secondary antibodies).

For the double tracing experiment the following two different combinations of antibodies were used.

In the first combination, primary antibodies were as follows: guinea-pig anti-porcine insulin (Dako, 1/400), chicken anti-GFP (Abcam, 1/400), mouse anti-mCherry (Abcam, 1/500), rabbit anti-human somatostatin (Dako, 1/200), and secondary antibodies were as follows: goat anti-guinea pig 647 (highly cross-adsorbed) (Molecular Probes, 1/500), goat anti-chicken 488 (Molecular Probes, 1/500), goat anti-mouse 555 (IgG1 -γ1) (Molecular Probes, 1/500), donkey anti-rabbit 594 (Molecular Probes, 1/500).

In the second combination, primary antibodies were as follows: guinea-pig anti-porcine insulin (Dako, 1/400), rabbit anti-GFP (Molecular Probes, 1/200), mouse anti-human somatostatin (BCBC (Ab1985), 1/200), and secondary antibodies were as follows: goat anti-guinea pig 647 (highly cross-adsorbed) (Molecular Probes, 1/500), goat anti-rabbit 488 (highly cross-adsorbed) (Molecular Probes, 1/500), goat anti-mouse TRITC (Molecular Probes, 1/500).

In the second combination, δ-cells were traced directly with the endogenous cell-expressed fluorophore, without further antibody amplification (1 hour 5% PFA for sample fixation).

Sections were observed under magnification with Leica TCS SPE, SP2 AOBS, Leica TCS SP5 STED CW confocal microscopes and Leica M205FA binocular equipped with a Leica DFC360FX camera, when appropriate. Section area quantifications were performed with Imaris or ImageJ programs.

### Physiological studies

Glucose tolerance tests and insulin dosages (immunoassay, ELISA kit mouse insulin ultrasensitive Mercodia) were performed as described in Thorel et al. (Nature, 2010, 464:1149-1154*).* Animals (4 males per group, 5-month-old) were fasted overnight for 12 hours before starting the experiment.

Insulin tolerance test was performed as described in Bonal et al. (Diabetes, 2013, 62:1443-1452*).* Animals (7 males for control and 10 males for DT-treated, 1.5-year-old) were fasted for 5 hours before the experiment. 0.75 U/kg per mice of Novorapid insulin was injected.

### Total RNA extraction, cDNA synthesis and qPCR

Adult and pup islets (n≥3) were isolated as described (Strom et al, 2007, Development 134, 2719-2725*)* and the samples were either directly processed for RNA extraction (1 sample per mouse) or incubated in accutase (Invitrogen) for 12 min at 37°C to prepare a single-cell suspension, followed by sorting on a FACSAria2 (BD Biosciences) or Moflo Astrios (Beckman Coulter) system (for β-cell sorting, 1 sample = 1 mouse; for δ-cell sorting, 1 sample = pool of 3 mice). For all samples the total RNA was isolated with the Qiagen RNeasy Micro kit (Qiagen #74004). The subsequent cDNA synthesis, qPCR reaction and data analysis, were performed either as described *(Thorel et al, 2010, supra*) or by using the RT2 Profiler PCR Array combined to RT (*Perl et al, 2002, supra*) SYBR Green ROX FAST Mastermix (QIAGEN) according to the manufacturer's instructions.

**Table 1. Primers sequences for gene amplification**

| **Gene** | **Primer Sequence** |
|---|---|
| **β-actin** | Forward primer: AAGGCCAACCGTGAAAAGAT (SEQ ID NO: 5) |
| | Reverse primer: GTGGTACGACCAGAGGGATAC (SEQ ID NO: 6) |
| **18S** | Forward primer: CAGATTGATGGCTCTTTCTCG (SEQ ID NO: 7) |
| | Reverse primer: AGACAAATCGCTCCACCAAC (SEQ ID NO: 8) |
| **AKT2** | Forward primer: AGGTAGCTGTCAACAAGGCA (SEQ ID NO: 9) |
| | Reverse primer: CTTGCCGAGGAGTTTGAGAT (SEQ ID NO: 10) |
| **AR** | Forward primer: CGAAGTGTGGTATCCTGGTG (SEQ ID NO: 11) |
| | Reverse primer: GGTACTGTCCAAACGCATGT (SEQ ID NO: 12) |
| **ARX** | Forward primer: TTTTCTAGGAGCAGCGGTGT (SEQ ID NO: 13) |
| | Reverse primer: AGTGGAAAAGAGCCTGCCAA (SEQ ID NO: 14) |
| **BRN4** | Forward primer: CATCGAGGTGAGTGTCAAGG (SEQ ID NO: 15) |
| | Reverse primer: CAGACACGCACCACTTCTTT (SEQ ID NO: 16) |
| **CDK2** | Forward primer: GGACTAGCAAGAGCCTTTGG (SEQ ID NO: 17) |
| | Reverse primer: AAGAATTTCAGGTGCTCGGT (SEQ ID NO: 18) |
| **CDKN1a** | Forward primer: AGTCTCATGGTGTGGTGGAA (SEQ ID NO: 19) |
| | Reverse primer: GACATCACCAGGATTGGACA (SEQ ID NO: 20) |
| **CDKN1b** | Forward primer: AGTGTCCAGGGATGAGGAAG (SEQ ID NO: 21) |
| | Reverse primer: CTTCTGTTCTGTTGGCCCTT (SEQ ID NO: 22) |
| **CDKN1c** | Forward primer: AATCAGCCAGCCTTCGAC (SEQ ID NO: 23) |
| | Reverse primer: ATCACTGGGAAGGTATCGCT (SEQ ID NO: 24) |
| **CKS1b** | Forward primer: TCCATGAACCAGAACCTCAC (SEQ ID NO: 25) |
| | Reverse primer: GGCTTCATTTCTTTGGCTTC (SEQ ID NO: 26) |
| **ESR1** | Forward primer: GCCTCAATGATGGGCTTATT (SEQ ID NO: 27) |
| | Reverse primer: AAAGCCTGGCACTCTCTTTG (SEQ ID NO: 28) |
| **FOXO1** | Forward primer: GAGAAGAGGCTCACCCTGTC (SEQ ID NO: 29) |
| | Reverse primer: ACAGATTGTGGCGAATTGAA (SEQ ID NO: 30) |
| **GADPH** | Forward primer: TCCATGACAACTTTGGCATTG (SEQ ID NO: 31) |
| | Reverse primer: CAGTCTTCTGGGTGGCAGTGA (SEQ ID NO: 32) |
| **GCG** | Forward primer: GAGGAGAACCCCAGATCATTCC (SEQ ID NO: 33) |
| | Reverse primer: TGTGAGTGGCGTTTGTCTTCA (SEQ ID NO: 34) |
| **GLUT2** | Forward primer: CTCGTGGCGCTGATGCT (SEQ ID NO: 35) |
| | Reverse primer: CTGGTTGAATAGTAAAATATCCCATTGA (SEQ ID NO: 36) |
| **Insulin2** | Forward primer: TCAACATGGCCCTGTGGAT (SEQ ID NO: 37) |
| | Reverse primer: AAAGGTGCTGCTTGAAAAAGC (SEQ ID NO: 38) |
| **MafA** | Forward primer: GGAGGTCATCCGACTGAAACA (SEQ ID NO: 39) |
| | Reverse primer: GCACCTCTCGCTCTCCAGAAT (SEQ ID NO: 40) |
| **MafB** | Forward primer: TGAGCTAGAGGGAGGAAGGA (SEQ ID NO: 41) |
| | Reverse primer: CCGGGTTTCTCTAACTCTGC (SEQ ID NO: 42) |
| **Ngn3** | Forward primer: GTCGGGAGAACTAGGATGGC (SEQ ID NO: 43) |
| | Reverse primer: GGAGCAGTCCCTAGGTATG (SEQ ID NO: 44) |
| **Nkx6**.**1** | Forward primer: AGAGAGCACGCTTGGCCTATTC (SEQ ID NO: 45) |
| | Reverse primer: GTCGTCAGAGTTCGGGTCCAG (SEQ ID NO: 46) |
| **Pax4** | Forward primer: GGACAAGGCTCCCAGTGTGT (SEQ ID NO: 47) |
| | Reverse primer: GCAAGCTCTGGTCTTCCTTGAA (SEQ ID NO: 48) |
| **PC 1/3** | Forward primer: TGGAGTTGCATATAATTCCAAAGTT (SEQ ID NO: 49) |
| | Reverse primer: CTAGCCTCAATGGCATCAGTT (SEQ ID NO: 50) |
| **Pdx1** | Forward primer: GCCCGGGTGTAGGCAGTAC (SEQ ID NO: 51) |
| | Reverse primer: CAGTGGGCAGGAGGTGCTTA (SEQ ID NO: 52) |
| **PDK1** | Forward primer: TAAAAGTTCAGACCTTTGGGCC (SEQ ID NO: 53) |
| | Reverse primer: TCCCGGCTCTGAATGGTG (SEQ ID NO: 54) |
| **SST** | Forward primer: CTCTCCCCCAAACCCCATAT (SEQ ID NO: 55) |
| | Reverse primer: TTTCTAATGCAGGGTCAAGTTGAG (SEQ ID NO: 56) |
| **SKP2** | Forward primer: GAAAGCTTCAGCTCTTTCCG (SEQ ID NO: 57) |
| | Reverse primer: AGGCCTTCCAGGCTTAGATT (SEQ ID NO: 58) |
| **Smad3** | Forward primer: GCACAGCCACCATGAATTAC (SEQ ID NO: 59) |
| | Reverse primer: GGAGGTAGAACTGGCGTCTC (SEQ ID NO: 60) |

For the second method, briefly, the relative expression of 84 genes of either the Hedgehog or the BMP/TGFβ pathways was evaluated using the PAMM-078Z (for the Hedgehog signaling pathway) and PAMM-035Z (for BMP/TGFβ Pathway). Samples were aliquot in the discs using the CorbettRobotics4 robot and the PCR reaction was performed in the CorbettResearch6000 series cycler using the RT² SYBR Green ROX FAST Mastermix (QIAGEN). CT values were exported from the qPCR instrument and analyzed with the ΔΔCt method using the online software provided by the manufacturer (http://pcrdataanalysis.sabiosciences.com/pcr/arrayanalysis.php). Five control genes, B2M, Hsp90ab1, Gusb, GAPDH, and β-actin present on the PCR array were used for normalization. For gene expression comparison between different age groups, the expression levels were always normalized to the appropriate age-matched controls, the difference in the expression levels reflecting solely the DT-effect on each age group.

### Statistical analyses

Statistical significance was assessed using Prism v6.0; the unpaired t-test with Welch's correction was applied.

### Example 1. Pancreatic δ-cells conversion into insulin producers occurs in young mice but not in adult mice

Total or near-total loss of β-cells is a situation found in diabetes (Type 1, T1D) (Atkinson, 2012, Cold Spring Harb Perspect Med; doi: 10.1101/cshperspect.a007641). It was previously observed that β-cell-ablated mice have the capability of reconstituting new insulin-producing cells in absence of autoimmunity *(*Thorel et al, 2010, Nature 464, 1149-1154*).* The process involves the contribution of islet non-β-cells; specifically, glucagon-producing α-cells begin producing insulin by a process of reprogramming (transdifferentiation) without proliferation.

There is evidence of efficient β-cell regeneration early in life in children or rodents with T1D or after pancreatic damage (*Thorel et al, 2010, supra).* The influence of age on β-cell reconstitution from heterologous islet cells after near-total β-cell loss was studied. It was found that α-cell plasticity is retained from puberty through adulthood. In contrast, prior to puberty, β-cell reconstitution is more efficient, always leading to diabetes recovery, and occurs through a newly identified mechanism: the spontaneous conversion of most somatostatin-producing δ-cells.

### 1.1. Prepubescent mice rapidly recover from diabetes after near-total β-cell loss

The regeneration potential during early postnatal life was studied by inducing β-cell ablation before weaning, at 2 weeks of age (Figure 1A). Mice received insulin treatment during 2.5 months following β-cell loss (Figure 1B). Four months after β-cell destruction all younglings were almost normoglycemic, thus displaying a faster recovery relative to adults (Figure1B). Accordingly, insulin transcripts were highly upregulated in pups as compared with adults at the same regeneration time-points (not shown).

Histologically, 99% of the β-cells were lost 2 weeks following DT administration. The insulin⁺ cell number had increased by a factor of 10 at 2 months of age, up to 9% of the normal age-matched β-cell mass. Subsequently, the insulin⁺ cell mass had increased 45-fold 4 months after ablation, representing 23% of the normal β-cell mass at 4.5 months of age. These values correlate with recovery of normoglycemia.

All animals remained normoglycemic during the rest of their life: in 4 mice euthanized at 15.5 months of age, insulin⁺ cell mass was on average 50% of the normal value at the corresponding age. Mice were neither intolerant to glucose nor insulin resistant during the period of analysis, up to 15 months after injury.

### 1.2. α-cells do not reprogram in pups

It was investigated whether the new insulin⁺ cells are reprogrammed α-cells, as previously observed in adults, using *Glucagon-rtTA; TetO-Cre; R26-YFP; RIP-DTR* multi-transgenic pups. Almost no insulin⁺ cell co-expressed YFP or glucagon in these pups (Table 2), indicating that insulin⁺ cell regeneration does not rely on α-cell plasticity, but on alternative mechanisms.

**Table 2**

| **YFP**⁺/**insulin**⁺ **(% cells ; 1.5 mpa)** | | | |
|---|---|---|---|
| Group | % | Standard Deviation | N (mice) |
| No DT (control) | 0.25 | +/- 0.1 | 4 |
| DT (2 week-old) | 0.23 | +/- 0.3 | 5 |

The age-dependency of rescue after near-total β-cell loss was further explored. To this aim, normoglycemic 5-month-old mice, which had recovered from near-total β-cell loss at 2 weeks of age, were re-administered DT to ablate the regenerated insulin⁺ cells. One month following the second massive ablation, 30% of the insulin⁺ cells also contained glucagon, like β-cell-ablated adults. The robust insulin⁺ cell regeneration displayed by pre-pubertal mice is therefore temporally restricted.

### 1.3. Increased islet cell proliferation after β-cell ablation in prepubescent mice

It was next explored if the newly formed insulin⁺ cells originate by replication of rare ablation-escaping β-cells. Proliferation rates were measured using anti-Ki67 antibody at different time-points during 2 months of regeneration. The proportion of Ki67-labeled insulin⁺ cells was very low, indicating that neither the escaping β-cells nor newly generated insulin⁺ cells enter the cell cycle during this initial period. However, there was a transient 3.5-fold increase in the number of insular Ki67⁺ cells 2 weeks after ablation. Such an increase was never observed in adult animals. Replicating cells were hormone-negative, chromogranin A-negative, and were not lineage-traced to either α- or escaping β-cells.

### 1.4. Regenerated insulin-producing cells are dedifferentiated δ-cells

Coincident with the peak of islet cell proliferation that followed β-cell destruction in pups, a 4.5-fold decrease in the number of somatostatin-producing δ-cells was observed (from 13 to 3 δ-cells/islet section), without indication of increased islet cell death. δ-cells were therefore lineage-traced in triply transgenic mice: *Somatostatin-Cre; R26-YFP; RIP-DTR.* In these animals, irreversible Cre-mediated labeling (i.e. YFP expression activation) allows the identification of δ-cells or their progeny even if somatostatin gene activity ceases. At 2 months of age, about 81% of δ-cells were YFP⁺ in the absence of β-cell ablation, whereas α-and β-cells were labeled at background levels (0.9% for β-cells and 0.2% for α-cells). During insulin⁺ cell reconstitution in pups, 2 weeks after β-cell ablation (i.e. in 1-month-old mice), 80% of YFP⁺ cells were proliferating (Ki67⁺) and somatostatin-negative, while most proliferating cells were YFP-labeled (85%). The increased islet cell proliferation observed in pups therefore specifically concerns dedifferentiated δ-cells.

Together, these observations suggest that in pre-pubertal mice most δ-cells undergo a loss of somatostatin expression and enter the cell cycle as a consequence of the massive ablation of β-cells.

The fate of proliferating dedifferentiated δ-cells was further investigated. At 2 months of age (i.e. 1.5 months post-ablation), most insulin⁺ cells expressed YFP (89%), indicating a δ-cell origin. Furthermore, in contrast to non-ablated age-matched controls in which, as expected, all YFP⁺ cells were somatostatin⁺ (99%), about half of YFP⁺ cells were insulin⁺ after 1.5 months of regeneration (45%), suggesting that about half of the progeny of dedifferentiated δ-cells had become insulin expressers. Bihormonal somatostatin⁺/insulin⁺ cells were rare. The observed increased number of insulin⁺ cells and δ-cell mass recovery at 1.5 month post DT is compatible with a single round of cell division from which, on average, 1 daughter cell becomes insulin expresser while the other re-expresses somatostatin.

It was confirmed with two other assays that regeneration and diabetes recovery in juvenile mice are δ-cell-dependent: by inducing β-cell destruction with streptozotocin (STZ) instead of DT, and by co-ablating β- and δ-cells simultaneously in *Somatostatin-Cre; R26-YFP; R26-iDTR* (Buch et al, 2005, Nature methods 2, 419-426); *RIP-DTR* mice. In absence of δ-cells, none of the mice recovered.

Interestingly, following β-cell ablation in adults it was found that δ-cells neither dedifferentiate nor proliferate: their numbers remain unchanged. Nevertheless, like α-cells, a few δ-cells reprogrammed into insulin production, so that after 1.5 month of regeneration, 17% of the very rare new insulin-producing cells were YFP⁺, i.e. δ-cell-derived.

### 1.5. Reconstituted insulin⁺ cells are new β-cells with transient proliferation capacity

Contrary to β-cells in age-matched adult mice, δ-cell-derived insulin⁺-cells replicated transiently; the insulin producing-cell mass thus reached between 30% to 69% of the normal β-cell values, and remained stable for life.

The δ-cell-derived insulin⁺ cells were further characterized at the gene expression level by qPCR. Islets isolated 2 weeks after β-cell ablation or after recovery (4 months post-DT) were first compared with age-matched control islets. Expression of all the β-cell-specific markers tested was robustly increased in recovered mice (Figure 2A). In order to further characterize this, regenerated insulin⁺ cells were compared with original β-cells using sorted mCherry⁺ cells obtained from either just recovered or unablated age-matched (4.5-month-old) *insulin-mCherry*, *RIP-DTR* mice (Figure 2B). The two cell populations were very similar (Figure 2C), yet δ-cell-derived replicating new insulin⁺ cells displayed a potent downregulation of cyclin-dependent kinase inhibitors and regulators (Figure 2D,E).

### 1.6. Ngn3 gene expression is upregulated after β-cell ablation in pre-pubertal mice

qPCR analyses on islets isolated from pups at different regeneration time-points revealed a transitory 5-fold upregulation of Ngn3 transcripts 6 weeks after β-cell ablation. Brief expression of Ngn3 is a feature of islet precursor cells in the embryonic pancreas *(*Desgraz and Herrera, 2009, Development 136, 3567-3574*).*

Ngn3 expression in the regenerating pancreas of pups, but not of ablated adults, was confirmed using *Ngn3-YFP; RIP-DTR* mice. Ngn3 transcriptional activity can be monitored in *Ngn3-YFP* transgenics because transient Ngn3 promoter activity results in transient YFP expression (Mellitzer et al. 2004, Molecular endocrinology 18, 2765-2776). In non-ablated age-matched control pups, or in ablated adults, no islet YFP⁺ cells were found (not shown). However, 6 weeks after ablation, 86% of the regenerated insulin⁺ cells coexpressed YFP, while 81% of YFP⁺ cells were insulin expressers. At 8 weeks of regeneration, YFP⁺ cells were not detectable.

These observations suggest that insulin⁺ cells originate from cells transiently activating Ngn3 expression after ablation.

This was confirmed with *Ngn3-Cre-ERT; R26-YFP; RIP-DTR* mice. In *Ngn3-CreERT* mice, tamoxifen (TAM) administration induces Cre activity in Ngn3-expressing cells (Gu et al, 2002, Development 129, 2447-2457). It was found that 91% of regenerated insulin⁺ cells were YFP-labeled, whereas 93% of YFP-labeled cells were insulin⁺ and no YFP⁺ cell contained somatostatin or glucagon.

### 1.7. δ-Cell de-differentiation, proliferation, commitment and new fate acquisition

The above observations are compatible with a model in which insulin⁺ cell reconstitution after ablation in younglings occurs following a defined sequence of events: δ-cells dedifferentiate, replicate once and then one half of the progeny activates Ngn3 expression before insulin production. This was tested in a combined double lineage-tracing experiment using *Somatostatin-Cre; R26-Tomato; Ngn3-YFP; RIP-DTR* mice. In this setting, δ-cells and their progeny are Tomato⁺, whereas Ngn3-expressing cells are YFP⁺. Six weeks post-β-cell ablation, nearly all insulin⁺ cells in younglings were Tomato⁺/YFP⁺, indicating that δ-cells sequentially dedifferentiate, proliferate, express Ngn3, and finally produce insulin (Figure 3).

### 1.7.1. δ-Cell transdifferentiation is Ngn3-dependent

It was explored whether preventing Ngn3 reactivation would affect insulin+ cell reconstitution. Mice bearing 5 mutant alleles were used: *Ngn3*-*tTA*^{+/+}*; TRE-Ngn3*^{+/+} *(*Wang et al, 2009, PNAS 106, 9715-9720); *RIP-DTR.* In these mice the Ngn3 coding region is replaced by a DOX-sensitive transactivator gene (tTA); the endocrine pancreas develops normally because Ngn3 expression is allowed in absence of DOX by the binding of tTA to the promoter of *TRE-Ngn3* transgene. Pups were given DT at 2 weeks of age and then DOX 2 weeks later, to block Ngn3 upregulation. They were euthanized when Ngn3 peaks after ablation (2-month-old; Figure 4A). It was found that insulin-expressing cells were more rare in regenerating islets lacking Ngn3 upregulation, and that they also contained glucagon (Figure 4B,C).

This observation suggests that Ngn3 activity is required for δ-to-insulin⁺ cell conversion in pups; if the reconstitution sequence is blocked, new insulin-expressing cells nevertheless emerge, but involving glucagon⁺/insulin⁺ bihormonal cells, similar to adult pancreas regeneration.

### Example 2. Differential gene regulation in juvenile and adult δ-cells after β-cell loss

It was then tried to determine the reason why δ-cells in prepubescent mice are able to fully reprogram and trans-fate, with de-differentiation and proliferation, but lose this plasticity in adulthood. Purified δ-cells were profiled before and after β-cell ablation, either before or after puberty, using *Somatostatin-Cre; R26-YFP; RIP-DTR* mice.

One key reprogramming and cell cycle entry player is FoxO1, a transcription factor whose downregulation triggers Ngn3 expression in human fetal pancreatic explants *(*Al-Masri et al, 2010, Diabetologia 53, 699-711*)* and favors insulin production in Ngn3⁺ entero-endocrine progenitors *(*Talchai et al, 2012, Nature genetics 44, 406-412*, S401).* FoxO1, in cooperation with TGFβ/SMAD signaling (Munoz-Espin et al, 2013, Cell 155, 1104-1118 *;* Seoane et al, 2004, Cell 117, 211-223*),* inhibits cell proliferation through the transcriptional regulation of cell cycle inhibitors and activators *(*Perk et al, 2005, Nature reviews. Cancer 5, 603-614*).* Briefly, the FoxO1 regulatory network involved in the regulation of cell cycle progression and cellular senescence comprises the following elements: FoxO1 arrests the cell cycle by repressing activators (cyclinD1, cyclinD2) and inducing inhibitors (cdkn1a/p21, cdkn1b/p27, cdkn2b/p15Ink4b, cdkn1c/p57). cdkn1a/p21 and cdkn2b/p15Ink4b activation, a sign of cellular senescence, is regulated by FoxO1 through direct interaction with Skp2 protein. In turn, Skp2 blocks FoxO1 and, together with CKS1b CDK1 and CDK2, triggers the direct degradation of cdkn1a/p21 and cdkn1b/p27, thus promoting proliferation. FoxO proteins are inhibited mainly through PI3K/AKT-mediated phosphorylation: PDK1, the master kinase of the pathway, stimulates cell proliferation and survival by directly activating AKT, which phosphorylates (inhibits) the FoxOs. PI3K/AKT/FoxO1 circuit requires active TGFβ/SMAD signaling in order to co-regulate cdkn1a/ p21-dependent cell senescence. Active TGFβ signaling also downregulates ID1 and ID2, two BMP pathway downstream effectors. ID 1 and ID2 are known to promote de-differentiation and proliferation during embryogenesis and cancer progression probably through cdkn2b/p15Ink4b regulation.

The FoxO1 molecular network was explored in purified adult or juvenile δ-cells shortly (1-week) after β-cell ablation, using *Somatostatin-Cre; R26-YFP; RIP-DTR* mice.

Interestingly, δ-cells displayed a divergent regulation of FoxO1 in injured juvenile and adult mice. Consistent with FoxO1 repression in δ-cells of pups, PDK1 and AKT levels were increased, cdkn1a/p21 and cdkn2b/p15Ink4b were downregulated, and CKS1b, CDK2 and SKP were upregulated (Figure 5A), thus explaining the proliferative capacity of juvenile δ-cells after β-cell ablation. The opposite was found in δ-cells of ablated adults (Figure 5A); of note, cdkn1a/p21 and cdkn1b/p27 were already increased in adult δ-cells as compared with juvenile ones, without β-cell injury, indicating a constitutive proliferation block in post-pubertal mice (not shown).

Moreover, in δ-cells of regenerating pups, but not in adults, there was a robust upregulation of BMP1/4 downstream effectors, such as ID1 (∼45-fold), ID2 (∼500-fold) and INHBA (~20-fold) (Figure 5B), which have been demonstrated to promote de-differentiation and proliferation during embryogenesis and cancer progression (Yokota, 2001, Oncogene 20, 8290-8298 *; Perk et al, 2005, supra*). Inversely, in δ-cells of regenerating adults, but not in juveniles, TGFβ pathway genes were upregulated (Figure 5B), in assent with the senescence scenario involving PI3K/FoxO1-TGFβ/SMAD cooperation to maintain differentiation and cycle arrest.

In summary, in δ-cells of regenerating pups, PI3K/AKT and SKP2/SCF pathways cooperate to inactivate FoxO1. Moreover, upregulation of BMP effectors (ID1 and ID2) contributes to δ-cell de-differentiation and proliferation. Conversely, the PI3K/AKT pathway remains blocked in δ-cells of ablated adults, thus FoxO1 is active and, together with TGFβ/SMAD effectors, inhibits proliferation and de-differentiation.

### Example 3. Induction of δ-cell conversion into insulin producing cells in diabetic adults

This situation was challenged in adult mice and it was tested whether FoxO1 downregulation would lead to a juvenile-like induction of δ-to-insulin⁺-cell conversion. Here, *Somatostatin-Cre; R26-YFP; RIP-DTR* β-cell-ablated diabetic adult mice were given a FoxO1 inhibitor compound (AS1842856) for 1 week, either immediately following ablation (Figure 5C) or 1 month after ablation (Figure 6E). While FoxO1 inhibition in non-ablated controls had minimal effect on insulin expression induction (Figure 6A, B, C & D), in DT-treated mice regeneration was improved with AS1842856: In mice where AS1842856 was administered immediately after DT ablation and analysed 1 month post ablation (1mpa), insulin⁺ cells were more abundant (11-fold; Figure 5D), and nearly all (93%) were YFP⁺, i.e. dedifferentiated δ-cells (Figure 5E). Conversely, one-fourth of the YFP⁺ cells expressed insulin only (Figure 5F), revealing that an important fraction of δ-cells had reprogrammed to insulin production, as in pups. A similar situation is observed when AS 1842856 is administered one month after DT ablation, with analysis performed 2mpa (Figure 6E, F, G & H)

These results show that inhibition of FoxO1 in β-cells ablated mice can induce conversion of δ-cells into insulin-producing cells. Considering that total or near-total loss of β-cells is a situation found in diabetes (Matveyenko et al, 2008, Diabetes, obesity & metabolism 10 Suppl 4, 23-31 *;* Atkinson 2012, Cold Spring Harbor perspectives in medicine 2*),* it derives that inhibition of FoxO1 in diabetic subjects could constitute a means for preventing/treating diabetes in those subjects.

### Example 4. Induction of δ-cell conversion into insulin producing cells in diabetic castrated adults

Three-week-old Somatostatin-Cre; R26-YFP; RIP-DTR transgenic male mice undergo bilateral orchiectomy and/or pharmacological castration as described earlier *(*Theve et al, 2008, Infect Immun. 76(9):4071-4078 *;* McDermott et al., 2012, Physiology and Behaviour 105(5):1168-1174). β -cell ablation is subsequently induced with DT later in life, at 10 weeks of age, so as to determine if in absence of sexual maturation δ -cells retain their plasticity for reprogramming into insulin-producing -cells. Moreover, 3-week-old host mice transplanted with adult islets are simultaneously castrated. Transgenic islets from adult donors (Somatostatin-Cre; R26-YFP; RIP-DTR, or Glucagon-Cre; R26-YFP; RIP-DTR) are transferred into wild-type immunodeficient SCID pre-pubertal 3-week-old hosts.

This experiment allows determining whether adult islets can rejuvenate and 6-cells can become plastic again.

### Sequence listing

SEQ ID NO: 1 (amino acid sequence of murine FoxO1)
MAEAPQVVETDPDFEPLPRQRSCTWPLPRPEFNQSNSTTSSPAPSGGAAANPDAAASLASASAVSTDFMS
NLSLLEESEDFARAPGCVAVAAAAAASRGLCGDFQGPEAGCVHPAPPQPPPTGPLSQPPPVPPSAAAAAG
PLAGQPRKTSSSRRNAWGNLSYADLITKAIESSAEKRLTLSQIYEWMVKSVPYFKDKGDSNSSAGWKNSI
RHNLSLHSKFIRVQNEGTGKSSWWMLNPEGGKSGKSPRRRAASMDNNSKFAKSRGRAAKKKASLQSGQEG
PGDSPGSQFSKWPASPGSHSNDDFDNWSTFRPRTSSNASTISGRLSPIMTEQDDLGDGDVHSLVYPPSAA
KMASTLPSLSEISNPENMENLLDNLNLLSSPTSLTVSTQSSPGSMMQQTPCYSFAPPNTSLNSPSPNYSK
YTYGQSSMSPLPQMPMQTLQDSKSSYGGLNQYNCAPGLLKELLTSDSPPHNDIMSPVDPGVAQPNSRVLG
QNVMMGPNSVMPAYGSQASHNKMMNPSSHTHPGHAQQTASVNGRTLPHVVNTMPHTSAMNRLTPVKTPLQ
VPLSHPMQMSALGSYSSVSSCNGYGRMGVLHQEKLPSDLDGMFIERLDCDMESIIRNDLMDGDTLDFNFD
NVLPNQSFPHSVKTTTHSWVSG

SEQ ID NO: 2 (amino acid sequence of human FoxO1)












SEQ ID NO: 3 (nucleic acid sequence of murine FoxO1)
AGGGGCGGGGCGGCGCGCGCCGCCGCGGGCGGGAGCCCGCTGCAGATCCCGTAAGACGGAGTCTGC
GGAGTCGCTTCAGTCCCCGCCGCCGCCACATTCAACAGGCAGCAGCGCCGCTGTCGCGCGGCCGCGGAGA
GCTAGAGCGGCCCGCAGCGTCCGCCCGTCTGCCTTGGCGTCCGCGGCCCTTGTCAGCGGGAGCGCGGTGC
CCGAGCTGCCGGGCTCCGCGGCCTGGTCGGTGCCCCGTCCTAGGCACGAACTCGGAGGCTCCTTAGACAC
CGGTGACCCAGCGAAGTTAAGTTCTGGGCGCGTCCGTCCGCTGCGCCCCGCCGCGCCTGACTCCGGCGTG
CGTCCGCCGTCCGCGGCCCCCCAATCTCGGAGCGACACTCGGGTCGCCCGCTCCGCGCCCCCGGTGGCCG
CGTCTCCCGGTACTTCTCTGCTGGTGGGGAGGGGCGGGGGCACCATGGCCGAAGCGCCCCAGGTGGTGG
AGACCGACCCGGACTTCGAGCCGCTGCCCCGGCAGCGCTCCTGTACCTGGCCGCTGCCCAGGCCGGAGTT
TAACCAGTCCAACTCGACCACCTCCAGTCCGGCGCCGTCGGGCGGCGCGGCCGCCAACCCCGACGCCGCG
GCGAGCCTGGCCTCGGCGTCCGCTGTCAGCACCGACTTTATGAGCAACCTGAGCCTGCTGGAGGAGAGTG
AGGACTTCGCGCGGGCGCCAGGCTGCGTGGCCGTGGCGGCGGCGGCTGCGGCCAGCAGGGGCCTGTGCGG
GGACTTCCAGGGCCCCGAGGCGGGCTGCGTGCACCCAGCGCCGCCACAGCCCCCACCGACCGGGCCGCTG
TCGCAGCCCCCACCCGTGCCTCCCTCCGCTGCCGCCGCCGCGGGGCCACTCGCGGGACAGCCGCGCAAGA
CCAGCTCGTCGCGCCGCAACGCGTGGGGCAACCTGTCGTACGCCGACCTCATCACCAAGGCCATCGAGAG
CTCAGCCGAGAAGAGGCTCACCCTGTCGCAGATCTACGAGTGGATGGTGAAGAGCGTGCCCTACTTCAAG
GATAAGGGCGACAGCAACAGCTCGGCGGGCTGGAAGAATTCAATTCGCCACAATCTGTCCCTTCACAGCA
AGTTTATTCGAGTGCAGAATGAAGGAACTGGAAAGAGTTCTTGGTGGATGCTCAATCCAGAGGGAGGCAA
GAGCGGAAAATCACCCCGGAGAAGAGCTGCGTCCATGGACAACAACAGTAAATTTGCTAAGAGCCGAGGG
CGGGCTGCTAAGAAAAAAGCATCTCTCCAGTCTGGGCAAGAGGGTCCTGGAGACAGCCCTGGGTCTCAGT
TTTCTAAGTGGCCTGCGAGTCCTGGGTCCCACAGCAACGATGACTTTGATAACTGGAGTACATTTCGTCC
TCGAACCAGCTCAAATGCTAGTACCATCAGTGGGAGACTTTCTCCCATCATGACAGAGCAGGATGACCTG
GGAGATGGGGACGTGCATTCCCTGGTGTATCCACCCTCTGCTGCCAAGATGGCGTCTACGCTGCCCAGTC
TGTCTGAAATCAGCAATCCAGAAAACATGGAGAACCTTCTGGATAATCTCAACCTTCTCTCGTCCCCAAC
ATCTTTAACTGTGTCCACCCAGTCCTCGCCTGGCAGCATGATGCAGCAGACACCATGCTATTCGTTTGCA
CCGCCAAACACCAGTCTAAATTCACCCAGTCCAAACTACTCAAAGTACACATACGGCCAATCCAGCATGA
GCCCTTTGCCCCAGATGCCTATGCAGACACTTCAGGACAGCAAATCAAGTTACGGAGGATTGAACCAGTA
TAACTGTGCCCCAGGACTCTTGAAAGAGTTGTTGACTTCTGACTCTCCTCCCCACAATGACATTATGTCA
CCGGTTGATCCCGGAGTGGCCCAACCCAACAGTCGGGTCCTGGGCCAAAATGTAATGATGGGCCCTAATT
CGGTCATGCCAGCGTATGGCAGCCAGGCATCTCATAACAAAATGATGAACCCCAGCTCCCACACCCACCC
TGGACATGCACAGCAAACGGCTTCGGTCAACGGCCGTACCCTGCCCCATGTGGTGAACACCATGCCTCAC
ACATCTGCCATGAACCGCTTGACCCCCGTGAAGACACCTTTACAAGTGCCTCTGTCCCACCCCATGCAGA
TGAGTGCCCTGGGCAGCTACTCCTCGGTGAGCAGCTGCAATGGCTATGGTAGGATGGGTGTCCTCCACCA
GGAGAAGCTCCCAAGTGACTTGGATGGCATGTTTATTGAGCGCTTGGACTGTGACATGGAGTCCATCATT
CGGAATGACCTCATGGATGGAGATACCTTGGATTTTAACTTTGATAATGTGTTGCCCAACCAAAGCTTCC
CACACAGTGTCAAGACTACAACACACAGCTGGGTGTCAGGCTAAGAGTTAGTGAGCAGGCTACATTTAAA
AGTCCTTCAGATTGTCTGACAGCAGGAACTGAGGAGCAGTCCAAAGATGCCCTTCACCCCTCCTTATAGT
TTTCAAGATTTAAAAAAAAAAAAAAAAAAAAAAAAAGTCCTTTCTCCTTTCCTCAGACTTGGCAACAGCG
GCAGCACTTTCCTGTGCAGGATGTTTGCCCAGCGTCCGCAGGTTTTGTGCTCCTGTAGATAAGGACTGTG
CCATTGGGAATCATTACAATGAAGTGCCAAACTCACTACACCATGTAATTGCAGAAAAGACTTTCAGATC
CTGGAGTGCTTTCAAGTTTTGTATATATGCAGTAGATACAGAATTGTATTTGTGTGTGTGTTTTTTAATA
CCTACTTGGTCCAAGGAAAGTTTATACTCTTTTGTAATACTGTGATGGTCTCAAGTCTTGATAAACTTTG
CTTTGTACTACCTGTGTTCTGCTACAGTGAGAAGTCATGAACTAAGATCTCTGTCCTGCACCTCGGCTGA
ATGACTGAACCTGGTCATTTGCCACAGAACCCATGAGAGCCAAGTAGCCAGTGATCAATGTGCTGAATTA
ATGGACTTGTCAAACTTTGGGGCAGAATAAGATTAAGTGCCAGCTTTGTACAGGTCTTTTTCTATTGTTT
TTGTTGTTGTTTATTTTGTTATTTGCAAATTTGTACAAACAACTTAAAATGGTTCTAATTTCCAGATAAA
TGACTTTTGATGTTATTGTTAGGACTCAACATCTTTTGGAATAGATACCGAAGTGTAATGTTTTCTTAAA
ACTAGAGTCTACTTTGTTACATTGTCTGCTTATAAATTTGTGAAATCAGAGGTATTTGGGGGCTGCATTC
ATAATTTTCATTTTGTATTTCTAACTGGATTAGTACTAATTTTATATGTGCTCAGCTGGTTTGTACACTT
TGCGATGATACCTGATAATGTTTCTGACTAATCGTAAACCATTGTAATTAGTACTTGCACACTCAACGTT
CCTGGCCCTTTGGGCAGGAAAGTTATGTATAGTTACAGACACTCTGTTTTGTGTGTAGATTTATGTGTGT
ATTTTAAAGAAATTTCACCTGCTTTTATTACCCTGTGAGTTGTGTACAGCGCATAGCACCAAGTCTTCAG
ATAGATGCCACGTGCTTACAGCCTTCTAGGGAAGCCTGCCAGATGATGCCCTGTGTCACGCTGTCATAGT

TCCCATGGAACTCTGTCTGTCGCTCAGGAAAGGGAACTTTATCTAAGGTGATGTTCTTTGTCTGACT
GGGGTTCGCCTCCTACTACTCTGAGCTGTTGGCTTTTGTCACGATGGAGGTGGCTTTGTGGCTCTGTCCT
GAAGAATCCTGTCACTTCTCGGTCCCCACCTCTGTTCTCTTTGGCTCTGAACAGTGTAAATCTAAGGAGG
AAGTTTACAAATAGGACTTCAGTGATTTATGGAGTGCTCTGTGCGCCTAAGTACAGACAGTGGCAGGATT
AGTTAAAAATGAAGGCAGTAAACTTGGAAACCAGCCAGCTATAAATGGACATTTATTTTGAAATCCTTAG
CTTAAGAATTTGAGAAGTTTTTCAGCCTTGAGCAGCCTAATGTGTCTCAAACATTTACGTTTTTTATAC
ATTCTATTTACCTGAAATCCTGCCAGACCAGGATAATTGGTTTTACCTCTCATTCCGTCCATCGGTGTTT
CCCAGTCTCCCACAGTTTGAGGAATAGATGTACCCCAGCACCCCTCTTTGCCTTTATGAGAAGGCCTGGT
TTGCATGAGAAGACCAAATTGCACTTCCATGAGAAGACCAAATTGTTTGTAGTGTTACTTAGCTCTCCCC
TCGTTTGTTAGTGTGTGTTAACAAGAATAAAATGTCCCTGCTTTCACCCACCGTTGGCCAGCTTTGTCAT
AGGCTTCCCACCATAACTTTCACTATTTAAACACATATTGAGCCACTGCTCGTCTGACTACCTTTGTTT
GGGCACTCCAAAACAGGACTTGTTTAGAAATGAACTCCTCCAAGTAGAGCCTCCTTCAAACAGAGTAGA
ATTTCCTGGTGTCAAAGAACCCGGGTCTGTCTCCCTTTCCTCCTCCCTCTGCCATTTCTTACCATTGCGG
AAAGAGAGAGCCTCCGTGTGTAATCATTCAGTAGAGGCAGCTACCGCCCTGGCAGTGGTCTACCTGCTGA
ATGCCACTGAATGACTAGGAGGTGTCTCTCCCTTCAGAAGCTGTCAATTTCAGCAGCAACCCCTGTTTTC
CTTGGTGTTAAGATCCCAGTGTGAATCATGGGCAGTTGTCTGGGGCACAGTGAACTCCAGGAAAGGCTTC
GTATCTGTTTTGAAAACAAACATCAAACGTGTGAGCTCCGAGGGTCCTTTTCTGGGAGAATGTTCGCTTT
CTGGTCTATTATTGTACATGATTGCTCTGTGAAAAGACTTCATCTATGCAGCCTTGTTTGATTCATTTCC
TTTGGTGTGTTCTGTTGTTAAGAGCAAATTGTATTATAGAGCTATTTGGATATTTTAAATATAAAGATGT
ATTGTTTCCATAATATAGATGTATGGAGTATATTTAGGTGATAGATGTACAACTTGGAAAGTTCTGCTTG
GACAAACTGAGTCTAAGTTAATTAGCAAATAATATATCCTGATGAGCAGGAAGCCCTGAAACCTAACAAC
AGTAAGCGGAGAAAATCACTTAAAATGGAAACAGTTCCCCAAAGGTGTTCAATTTGAACTTGTTCAACTG
CTTAATATATGGTCCCCCCCCCCCCAAAAAAAAAACCTTGAAGTTCTTAGTTTTCAGCTCTCCAAGTTA
CTGATTTTAAGTGAAGTTTCTCTGTGGTTTCAGCTGGGGAGTGATTGTTCAGTAGAGTGTGCATTGTGCT
TTATGCAAACCAAACAGCCTGGCCCTGTGGCCGGGGACAGACAGACAGCCCGTCAGGATAGAGTCCCGCC
CTTCGCCACCACAGCGGACTTGAGTAACAGTGCAGATGCCTTGCTCCTGTTCCATTGCTATCTGAGAAGT
GCCTGATGAGGATGGTAAACTTACAGACACAAGAACAATCCTTACTGTGCGTTGTATAAAGCCATAAATG
TACATAAATCATCTTAAGTGGC

SEQ ID NO: 4 (nucleic acid sequence of human FoxO1)
GCAGCCGCCACATTCAACAGGCAGCAGCGCAGCGGGCGCGCCGCTGGGGAGAGCAAGCGGCCCGCGGCGT
CCGTCCGTCCTTCCGTCCGCGGCCCTGTCAGCTGGAGCGCGGCGCAGGCTCTGCCCCGGCCCGGCGGCTC
TGGCCGGCCGTCCAGTCCGTGCGGCGGACCCCGAGGAGCCTCGATGTGGATGGCCCCGCGAAGTTAAGTT
CTGGGCTCGCGCTTCCACTCCGCCGCGCCTTCCTCCCAGTTTCCGTCCGCTCGCCGCACCGGCTTCGTTC
CCCCAAATCTCGGACCGTCCCTTCGCGCCCCCTCCCCGTCCGCCCCCAGTGCTGCGTTCTCCCCCTCTTG
GCTCTCCTGCGGCTGGGGGAGGGGCGGGGGTCACCATGGCCGAGGCGCCTCAGGTGGTGGAGATCGACCC
GGACTTCGAGCCGCTGCCCCGGCCGCGCTCGTGCACCTGGCCGCTGCCCAGGCCGGAGTTTAGCCAGTCC
AACTCGGCCACCTCCAGCCCGGCGCCGTCGGCAGCGCGGCTGCCAACCCCGACGCCGCGGCGGGCCTGC
CCTCGGCCTCGGCTGCCGCTGTCAGCGCCGACTTCATGAGCAACCTGAGCTTGCTGGAGGAGAGCGAGGA
CTTCCCGCAGGCGCCCGGCTCCGTGGCGGCGGCGGTGGCGGCGGCGGCCGCCGCGGCCGCCACCGGGGGG
CTGTGCGGGGACTTCCAGGGCCCGGAGGCGGGCTGCCTGCACCCAGCGCCACCGCAGCCCCCGCCGCCCG
GGCCGCTGTCGCAGCACCCGCCGGTGCCCCCCGCCGCCGCTGGGCCGCTCGCGGGGCAGCCGCGCAAGAG
CAGCTCGTCCCGCCGCAACGCGTGGGGCAACCTGTCCTACGCCGACCTCATCACCAAGGCCATCGAGAGC
TCGGCGGAGAAGCGGCTCACGCTGTCGCAGATCTACGAGTGGATGGTCAAGAGCGTGCCCTACTTCAAGG
ATAAGGGTGACAGCAACAGCTCGGCGGGCTGGAAGAATTCAATTCGTCATAATCTGTCCCTACACAGCAA
GTTCATTCGTGTGCAGAATGAAGGAACTGGAAAAAGTTCTTGGTGGATGCTCAATCCAGAGGGTGGCAAG
AGCGGAAAATCTCCTAGGAGAAGAGCTGCATCCATGGACAACAACAGTAAATTTGCTAAGAGCCGAAGCC
GAGCTGCCAAGAAGAAAGCATCTCTCCAGTCTGGCCAGGAGGGTGCTGGGGACAGCCCTGGATCACAGTT
TTCCAAATGGCCTGCAAGCCCTGGCTCTCACAGCAATGATGACTTTGATAACTGGAGTACATTTCGCCCT
CGAACTAGCTCAAATGCTAGTACTATTAGTGGGAGACTCTCACCCATTATGACCGAACAGGATGATCTTG
GAGAAGGGGATGTGCATTCTATGGTGTACCCGCCATCTGCCGCAAAGATGGCCTCTACTTTACCCAGTCT
GTCTGAGATAAGCAATCCCGAAAACATGGAAAATCTTTTGGATAATCTCAACCTTCTCTCATCACCAACA
TCATTAACTGTTTCGACCCAGTCCTCACCTGGCACCATGATGCAGCAGACGCCGTGCTACTCGTTTGCGC
CACCAAACACCAGTTTGAATTCACCCAGCCCAAACTACCAAAAATATACATATGGCCAATCCAGCATGAG
CCCTTTGCCCCAGATGCCTATACAAACACTTCAGGACAATAAGTCGAGTTATGGAGGTATGAGTCAGTAT
AACTGTGCGCCTGGACTCTTGAAGGAGTTGCTGACTTCTGACTCTCCTCCCCATAATGACATTATGACAC
CAGTTGATCCTGGGGTAGCCCAGCCCAACAGCCGGGTTCTGGGCCAGAACGTCATGATGGGCCCTAATTC
GGTCATGTCAACCTATGGCAGCCAGGCATCTCATAACAAAATGATGAATCCCAGCTCCCATACCCACCCT
GGACATGCTCAGCAGACATCTGCAGTTAACGGGCGTCCCCTGCCCCACACGGTAAGCACCATGCCCCACA
CCTCGGGTATGAACCGCCTGACCCAAGTGAAGACACCTGTACAAGTGCCTCTGCCCCACCCCATGCAGAT

GAGTGCCCTGGGGGCTACTCCTCCGTGAGCAGCTGCAATGGCTATGGCAGAATGGGCCTTCTCCACCAG
GAGAAGCTCCCAAGTGACTTGGATGGCATGTTCATTGAGCGCTTAGACTGTGACATGGAATCCATCATTC
GGAATGACCTCATGGATGGAGATACATTGGATTTTAACTTTGACAATGTGTTGCCCAACCAAAGCTTCCC
ACACAGTGTCAAGACAACGACACATAGCTGGGTGTCAGGCTGAGGGTTAGTGAGCAGGTTACACTTAAAA
GTACTTCAGATTGTCTGACAGCAGGAACTGAGAGAAGCAGTCCAAAGATGTCTTTCACCAACTCCCTTTT
AGTTTTCTTGGTTAAAAAAAAAAACAAAAAAAAAAACCCTCCTTTTTCCTTTCGTCAGACTTGGCAGCA
AAGACATTTTTCCTGTACAGGATGTTTGCCCAATGTGTGCAGGTTATGTGCTGCTGTAGATAAGGACTGT
GCCATTGGAAATTTCATTACAATGAAGTGCCAAACTCACTACACCATATAATTGCAGAAAAGATTTTCAG
ATCCTGGTGTGCTTTCAAGTTTTGTATATAAGCAGTAGATACAGATTGTATTTGTGTGTGTTTTTGGTTT
TTCTAAATATCCAATTGGTCCAAGGAAAGTTTATACTCTTTTTGTAATACTGTGATGGGCCTCATGTCTT
GATAAGTTAAACTTTTGTTTGTACTACCTGTTTTCTGCGGAACTGACGGATCACAAAGAACTGAATCTCC
ATTCTGCATCTCCATTGAACAGCCTTGGACCTGTTCACGTTGCCACAGAATTCACATGAGAACCAAGTAG
CCTGTTATCAATCTGCTAAATTAATGGACTTGTTAAACTTTTGGAAAAAAAAAGATTAAATGCCAGCTTT
GTACAGGTCTTTTCTATTTTTTTTTGTTTATTTTGTTATTTGCAAATTTGTACAAACATTTAAATGGTTC
TAATTTCCAGATAAATGATTTTTGATGTTATTGTTGGGACTTAAGAACATTTTTGGAATAGATATTGAAC
TGTAATAATGTTTTCTTAAAACTAGAGTCTACTTTGTTACATAGTCAGCTTGTAAATTTTGTGGAACCAC
AGGTATTTGGGGCAGCATTCATAATTTTCATTTTGTATTCTAACTGGATTAGTACTAATTTTATACATGC
TTAACTGGTTTGTACACTTTGGGATGCTACTTAGTGATGTTTCTGACTAATCTTAAATCATTGTAATTAG
TACTTGCATATTCAACGTTTCAGGCCCTGGTTGGGCAGGAAAGTGATGTATAGTTATGGACACTTTGCGT
TTCTTATTTAGGATAACTTAATATGTTTTTATGTATGTATTTTAAAGAAATTTCATCTGCTTCTACTGAA
CTATGCGTACTGCATAGCATCAAGTCTTCTCTAGAGACCTCTGTAGTCCTGGGAGGCCTCATAATGTTTG
TAGATCAGAAAAGGGAGATCTGCATCTAAAGCAATGGTCCTTTGTCAAACGAGGGATTTTGATCCACTTC
ACCATTTTGAGTTGAGCTTTAGCAAAAGTTTCCCCTCATAATTCTTTGCTCTTGTTTCAGTCCAGGTGGA
GGTTGGTTTTGTAGTTCTGCCTTGAGGAATTATGTCAACACTCATACTTCATCTCATTCTCCCTTCTGCC
CTGCAGATTAGATTACTTAGCACACTGTGGAAGTTTAAGTGGAAGGAGGGAATTTAAAAATGGGACTTGA
GTGGTTTGTAGAATTTGTGTTCATAAGTTCAGATGGGTAGCAAATGGAATAGAACTTACTTAAAAATTGG
GGAGATTTATTTGAAAACCAGCTGTAAGTTGTGCATTGAGATTATGTTAAAAGCCTTGGCTTAAGAATTT
GAAAATTTCTTTAGCCTGTAGCAACCTAAACTGTAATTCCTATCATTATGTTTTATTACTTTCCAATTAC
CTGTAACTGACAGACCAAATTAATTGGCTTTGTGTCCTATTTAGTCCATCAGTATTTTCAAGTCATGTGG
AAAGCCCAAAGTCATCACAATGAAGAGAACAGGTGCACAGCACTGTTCCTCTTGTGTTCTTGAGAAGGAT
CTAATTTTTCTGTATATAGCCCACATCACACTTGCTTTGTCTTGTATGTTAATTGCATCTTCATTGGCTT
GGTATTTCCTAAATGTTTAACAAGAACACAAGTGTTCCTGATAAGATTTCCTACAGTAAGCCAGCTCTAT
TGTAAGCTTCCCACTGTGATGATCATTTTTTTGAAGATTCATTGAACAGCCACCACTCTATCATCCTCAT
TTTGGGGCAGTCCAAGACATAGCTGGTTTTAGAAACCCAAGTTCCTCTAAGCACAGCCTCCCGGGTATGT
AACTGAACTTGGTGCCAAAGTACTTGTGTACTAATTTCTATTACTACGTACTGTCACTTTCCTCCCGTGC
CATTACTGCATCATAATACAAGGAACCTCAGAGCCCCCATTTGTTCATTAAAGAGGCAACTACAGCCAAA
ATCACTGTTAAAATCTTACTACTTCATGGAGTAGCTCTTAGGAAAATATATCTTCCTCCTGAGTCTGGGT
AATTATACCTCTCCCAAGCCCCCATTGTGTGTTGAAATCCTGTCATGAATCCTTGGTAGCTCTCTGAGAA
CAGTGAAGTCCAGGGAAAGGCATCTGGTCTGTCTGGAAAGCAAACATTATGTGGCCTCTGGTAGTTTTT
TCCTGTAAGAATACTGACTTTCTGGAGTAATGAGTATATATCAGTTATTGTACATGATTGCTTTGTGAAA
TGTGCAAATGATATCACCTATGCAGCCTTGTTTGATTTATTTTCTCTGGTTTGTACTGTTATTAAAAGCA
TATTGTATTATAGAGCTATTCAGATATTTTAAATATAAAGATGTATTGTTTCCGTAATATAGACGTATGG
AATATATTTAGGTAATAGATGTATTACTTGGAAAGTTCTGCTTTGACAAACTGACAAAGTCTAAATGAGC
ACATGTATCCCAGTGAGCAGTAAATCAATGGAACATCCCAAGAAGAGGATAAGGATGCTTAAAATGGAAA
TCATTCTCCAACGATATACAAATTGGACTTGTTCAACTGCTGGATATATGCTACCAATAACCCCAGCCCC
AACTTAAAATTCTTACATTCAAGCTCCTAAGAGTTCTTAATTTATAACTAATTTTAAAAGAGAAGTTTCT
TTTCTGGTTTTAGTTTGGGAATAATCATTCATTAAAAAAAATGTATTGTGGTTTATGCGAACAGACCAAC
CTGGCATTACAGTTGGCCTCTCCTTGAGGTGGGCACAGCCTGGCAGTGTGGCCAGGGGTGGCCATGTAAG
TCCCATCAGGACGTAGTCATGCCTCCTGCATTTCGCTACCCGAGTTTAGTAACAGTGCAGATTCCACGTT
CTTGTTCCGATACTCTGAGAAGTGCCTGATGTTGATGTACTTACAGACACAAGAACAATCTTTGCTATAA
TTGTATAAAGCCATAAATGTACATAAATTATGTTTAAATGGCTTGGTGTCTTTCTTTTCTAATTATGCAG
AATAAGCTCTTTATTAGGAATTTTTTGTGAAGCTATTAAATACTTGAGTTAAGTCTTGTCAGCCACAA

### Primers for PCR: SEQ ID NO: 5 to 60: see Table 1

## Claims

1. An *ex-vivo* method of inducing insulin production in δ-cells and/or converting δ-cells into insulin producing cells, comprising the steps of:
- obtaining a population of δ-cells from a subject, in particular a mammalian subject;
- contacting, *ex vivo,* said population of δ-cells with at least one FOXO1 inhibitor, thereby generating insulin-producing cells;
- optionally, collecting said insulin-producing cells.

2. The method according claim 1, wherein said FOXO1 inhibitor is a small molecule selected from the group consisting of: 5-amino-7-(cyclohexylamino)-1-ethyl-6-fluoro-4-oxo-1,4-dihydroquinoline-3-carboxylic acid (AS 1842856), 1-cyclopentyl-6-fluoro-4-oxo-7-(tetrahydro-2H-pyran-3-ylamino)-1,4-dihydro-quinoline-3-carboxylic acid (AS 1841674), 7-(cyclohexylamino)-6-fluoro-4-oxo-1-(prop-1-en-2-yl)-1,4-dihydroquinoline-3-carboxylic acid (AS1838489), 7-(cyclohexylamino)-6-fluoro-1-(3-fluoroprop-1-en-2-yl)-4-oxo-1,4-dihydroquinoline-3-carboxylic acid (AS 1837976), 7-(cyclohexylamino)-1-(cyclopent-3-en-1-yl)-6-fluoro-4-oxo-1,4-dihydro-quinoline-3-carboxylic acid (AS1805469) and 7-(cyclohexylamino)-6-fluoro-5-methyl-4-oxo-1-(pentan-3-yl)-1,4-dihydroquinoline-3-carboxylic acid (AS1846102).

3. The method according to any one of claims 1 to 2, wherein said FOXO1 inhibitor is 5-amino-7-(cyclohexylamino)-1-ethyl-6-fluoro-4-oxo-1,4-dihydroquinoline-3-carboxylic acid of formula:

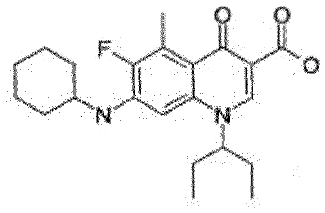

4. The method according to any one of claims 1 to 3, wherein said δ-cells are gastrointestinal δ-cells from a subject suffering from, or at risk of suffering from, diabetes.

5. A FOXO1 inhibitor targeting pancreatic islets or δ-cells comprising a FOXO1 inhibitor and a ligand directed to a pancreatic islet or a δ-cell specific marker.

6. A FOXO1 inhibitor according to claim 5, comprising a FOXO1 inhibitor loaded into a nanoparticle, liposome or nanotube, comprising a surface ligand directed to a pancreatic islet or δ-cell specific marker.

7. A FOXO1 inhibitor according to any one of claims 5 to 6, wherein said inhibitor comprises 5-amino-7-(cyclohexylamino)-1-ethyl-6-fluoro-4-oxo-1,4-dihydroquinoline-3-carboxylic acid of formula:

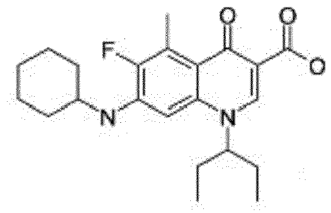

8. Isolated δ-cells converted into insulin producing cells obtainable by the method according to any one of claims 1 to 4.

9. Isolated δ-cells according to claim 8, wherein said cells are from a subject suffering from, or at risk of suffering from, diabetes, or from a subject not at risk of suffering diabetes.

10. A composition comprising at least one FOXO1 inhibitor targeting pancreatic islets or δ-cells according to any one of claims 5 to 7 and/or isolated δ-cells converted into insulin producing cells according to any one of claims 8 to 9.

11. A pharmaceutical composition comprising at least one FOXO1 inhibitor targeting pancreatic islets or δ-cells according to any one of claims 5 to 7 and/or isolated δ-cells converted into insulin producing cells according to any one of claims 8 to 9, and a pharmaceutically acceptable excipient.

12. A FOXO1 inhibitor targeting pancreatic islets or δ-cells according to any one of claims 5 to 7 for use as a medicament.

13. A FOXO1 inhibitor targeting pancreatic islets or δ-cells according to any one of claims 5 to 7 for use in preventing and/or treating diabetes.

14. Isolated δ-cells, optionally within isolated pancreatic islets, converted into insulin producing cells according to any one of claims 8 to 9 for use as a medicament.

15. Isolated δ-cells, optionally within isolated pancreatic islets, converted into insulin producing cells according to any one of claims 8 to 9 for use in preventing and/or treating diabetes.
